# EUROPEAN PATENT APPLICATION

(11) **EP 4 599 821 A1**
(43) Date of publication of application: **13.08.2025**
(21) Application number: 24156158.8
(22) Date of filing: 06.02.2024
(51) Int. Cl.: A61K 9/127, A61K 31/00, A61K 47/14

(54) **CANNABIDIOL-COMPRISING LIPOSOMES**

(71) Applicant: Novozymatic BV, 2950 Kapellen (BE)
(72) Inventor: KWIECINSKI, Wlodzimierz, 2950 Kapellen (BE); SPIRA, Jack, 2950 Kapellen (BE); RISCHER, Matthias, 60388 Frankfurt (DE); MOHR, Wolfgang, 91183 Abenberg (DE); KOPTON, RAMONA, 79106 Freiburg i. Br. (DE)
(74) Representative: Hoffmann Eitle

(57) **Abstract**

The present invention pertains to a liposome comprising cannabidiol (CBD), a phospholipid component and optionally a sterol component, wherein the liposome comprises more than one bilayer, to related mixtures of liposomes, processes for manufacturing, medical uses and compositions.

## Description

The present invention relates to means for the delivery of a pharmaceutically active substance. More specifically, it relates inter alia to a liposome comprising cannabidiol (CBD) and to processes for manufacturing liposomes comprising CBD.

Cannabinoids are lipophilic and potentially acid-labile compounds. Because of their hydrophobic nature, cannabinoids are poorly absorbed systemically from oral dosage forms because of the poor dissolution of cannabinoids in the aqueous environment of gastrointestinal tract. Oral formulations of cannabinoids, therefore, exhibit low bioavailability.

The amount of cannabinoids reaching the blood stream by absorption through the digestive system is only 5-10% of the administered dose. Fasting or food deprivation may further decrease the rate of absorption of cannabinoids. Inhaled CBD was reported to have an average systemic bioavailability of 31% (Lucas et al. (2018) Br J Clin Pharmacol 84, 2477-2482).

Most preparations of cannabinoids contain a mixture of tetrahydrocannabinol (THC) and cannabidiol (CBD). An example is Sativex^{®}, a mouth spray (oro-mucosal) containing tetrahydrocannabinol (THC) and cannabidiol (CBD). It is approved for the treatment of spasticity due to multiple sclerosis. Administration of synthetic cannabinoid formulations show fewer undesirable side effects than THC. Sativex reaches its Cmax after 45-120 minutes after administration. The Cmax and AUC are highly variable and change (increase) following food intake. As an example of this, the amount of CBD that is measured following 9 days of dosing varies in the Cmax interval of 0.34 -3.39 ng/ml and in the AUC interval (AUC0-τ) of 2.40 -13.19 hr*ng/ml. Hence, although oro-mucosal delivery has the advantage of rapid absorption via the oral mucosa and hence to be useful for symptoms requiring rapid relief, it produces plasma drug concentrations higher relative to oral, but reduced relative to inhaled THC. However, part of the dose may be swallowed and orally absorbed and as seen above the variability is considerable.

Epidyolex is a preparation of pure cannabidiol (CBD) that is commercially available as a solution for oral (gastrointestinal) delivery. Following a single dose, Cmax is reached after 2.5 to 5 hrs. The absolute bioavailability of oral CBD is low (6.49%) following oral administration in fasting conditions. The uptake is highly dependent on simultaneous intake of fat-rich food or alcohol. Food intake was shown to impact the relative bioavailability of CBD with a positive 3-fold increase when CBD was taken with a high-fat meal. Due to significant food effect observed, the bioavailability following administration with food can be expected around 14-25% (European Medicines Agency Assessment Report, see https://www.ema.europa.eu/en/documents/assessment-report/epidyolex-epar-public-assessment-report en.pdf). The therapeutic indications are seizures associated with Lennox-Gastaut syndrome (LGS) or Dravet syndrome (DS),

The bioavailability of CBD varies greatly with route and mode of administration. Due to the highly lipophilic nature of CBD (Log P 6.3), it is most commonly supplied as an oil or alcoholic formulation either in soft-gel capsules, liquid solution, sublingual drops, or as an oromucosal spray. Highly lipophilic CBD delivered orally in solution can precipitate in the gastrointestinal (GI) tract, resulting in an absorption rate slower than elimination. As stated above, the oral bioavailability of CBD is low. Time to peak plasma concentration following oral delivery is slow (1-4 h), the Cmax (maximum concentration) generated from 20 mg of orally delivered CBD/THC sprayed onto a gelatin capsule was 2.4 ng/mL of CBD. Because of their poor absorption, large interindividual variation and poor bioavailability, oral formulations have the additional disadvantage that they might require several administrations a day, making it inconvenient for patients who have difficulty swallowing. After oromucosal spray, the half-life of CBD was reported between 1.4 and 10.9 h.

CBD has substantial therapeutic potential, but its development as an effective drug by the pharmaceutical industry is hindered by intrinsic characteristics such as the mentioned low and variable bioavailability, low water solubility, and variable pharmacokinetic profiles. Potential avenues to overcome these issues with CBD include self-emulsifying drug delivery systems, improved crystal formulations and other solid-state delivery formulations, which are mostly in the pre-clinical or early clinical stages of development.

CBD interacts with the endocannabinoid system (ECS), which plays a crucial role in regulating various physiological processes, e.g. sleep, appetite, pain, immune function and nervous system-derived pathologies. CBD has been implicated in reducing anxiety or depression and improving mood. It is therapeutically used in a number of diseases such as Alzheimer's disease/dementia, glaucoma, traumatic brain injury/spinal cord injury, chemotherapy-induced nausea/vomiting, appetite and weight loss, chronic pain, cancer, Huntington's disease, Parkinson's disease, dystonia, addiction, anxiety, depression, irritable bowel syndrome, epilepsy, spasticity of multiple sclerosis, Tourette syndrome, amyotrophic lateral sclerosis, sleep disorders, posttraumatic stress disorder, and schizophrenia.

In order to be successfully utilised as a medicine, it is paramount to identify and overcome the inherent challenges that face CBD's effective delivery, particularly through the oral route, which is the most preferred route for drug delivery by patients.

Nanoliposomes are bilayer lipid vesicles possessing and maintaining nanometric size. They have been referred to as nanoscale bilayer lipid vesicles. In relation to microencapsulation techniques viz, polymeric, chitosan and alginate based carriers, lipid based nanoencapsulation possess unique distinguished advantages, including the enhanced ability to entrap material with various range of solubility. Liposomes are formed especially from phospholipids, which contain hydrophilic and hydrophobic parts and thus have amphiphilic properties. This unique property of phospholipids tends to improve self-sealing characteristics. Known phospholipids are e.g. phosphatidylserine, phosphatidylcholine, phosphatidylinositol, phosphatidylglycerol and phosphatidic acid.

Various methods for the manufacture of nanoliposomes are known, for instance the thinfilm hydration sonication (Bangham) method, ethanol injection, reverse phase evaporation, supercritical fluid technology (e.g. SAS and RESS), supercritical assisted liposome formation (SuperLip), depressurisation of an expanded liquid organic solution (DELOS-SUSP), particles from gas saturated solution (PGSS), depressurization of an expanded solution into aqueous media (DESAM), the heating method and the Mozafari method.

Usually, the goal in the art is to achieve small unilamellar liposomes with a hydrodynamic diameter ≤ 300 nm and a homogenous dispersion, PDI ≤ 0.3. This goal is based upon the idea that liposomes with these characteristics will provide optimal bioavailability (see e.g. Hoseini et al. (2023) Scientific Reports 13, 18012). In line with this, the majority of commercial liposomal products are small unilamellar liposomes in this size range, often even having a size of at most 150 nm or at most 100 nm. Moreover, large size of liposomes is often regarded as a problem for oral delivery of a liposomal drug because it is perceived to result in instability in the gastrointestinal tract and poor permeability across the intestinal epithelia.

The unsatisfactory bioavailability of cannabidiol (CBD) still represents an unmet need in the art. It is thus the object of present invention to provide a means for the administration of cannabidiol (CBD) which allows for improved CBD bioavailability. Preferably, this means has a better oral bioavailability than current oral CBD formulations. Advantageously, the means has a beneficial property other than bioavailability, such as improved dissolution, enhanced absorption or less interindividual variability.

According to a first aspect of the present invention, this object is solved by a liposome comprising cannabidiol (CBD), a phospholipid component and optionally a sterol component, wherein the liposome comprises more than one bilayer.

It has been found that surprisingly, a higher bioavailability of CBD is observed if there is an increased amount and size of CBD-comprising liposomes with more than one bilayer (such as MLV-MVVs, multilamellar-multivesicular liposomes) and/or a higher polydispersity index. This departs from conventional goals in liposome technology, which favour smaller over larger liposomes, a narrow over a broader size distribution and unilamellar liposomes over those with more than one bilayer.

Thus, the CBD-comprising liposome according to the invention, which comprises more than one bilayer, is advantageous for CBD bioavailability.

The liposome according to the invention comprises at least one phospholipid (i.e., one or more phospholipids) and optionally at least one sterol (i.e., one or more sterols).

Preferably, the liposome according to the invention fulfils one or more of the following conditions:
a) the phospholipid component comprises or consists of phosphatidylcholine, wherein the phosphatidylcholine is preferably 1,2-dimyristoyl-sn-glycero-3-phosphocholine (DMPC),
b) the liposome comprises a sterol component which comprises or consists of cholesterol,
c) the liposome does not comprise a sterol component,
d) the ratio of the weight of CBD to the total weight of the phospholipid component and, if present, the sterol component (CBD:lipid ratio) is 0.25, 0.42, 0.5, 0.7, 0.8, 1.0, 1.3, from 0.1 to 9, from 0.1 to 4, from 0.1 to 2.3, from 0.1 to 1.3, from 0.25 to 1.3, from 0.42 to 1.0 or from 0.5 to 0.8, wherein the CBD:lipid ratio is preferably 0.7,
e) the liposome is substantially free from tetrahydrocannabinol,
f) a combination of the above a) and b),
g) a combination of the above a) and b), wherein the weight ratio of the phospholipid component to the sterol component is preferably from 70:30 to 90:10, more preferably from 75:25 to 85:15 and most preferably 80:20,
h) a combination of the above a) and c),
i) a combination of the above a), b) and d),
j) a combination of the above a), c) and d),
k) a combination of the above a), b), d) and e), or
l) a combination of the above a), c), d) and e).

Herein, unless stated otherwise, a reference to the "lipid", as such or in terms like "CBD:lipid ratio", "lipid composition" and the like, refers collectively to the phospholipid component and, if any, the sterol component.

As to the above features a) and b), the option "consists of" is preferred. Particularly preferred is a combination of a phospholipid component that consists of DMPC and a sterol component that consists of cholesterol.

In the above feature d), as to the weight of the phospholipid component, if there is only one phospholipid, it means the weight of the phospholipid, if there is more than one phospholipid, it means the sum of the weights of all phospholipids. As to the weight of the sterol component, if there is a sterol and it is only one sterol, it means the weight of the sterol, if there is more than one sterol, it means the sum of the weights of all sterols. In any event, as specified, for the lipid in the CBD:lipid ratio, the sum of the weight of the phospholipid component and, if present, the sterol component is considered.

Preferably, the liposome according to the invention is selected from the group consisting of a bilamellar liposome (BLV), an oligolamellar liposome (OLV), a multilamellar liposome (MLV), a multivesicular liposome (MVV) and a multilamellar-multivesicular liposome (MLV-MVV). As used herein, the types of liposomes are defined as follows:
- unilamellar (ULV, liposome with a single bilayer),
- bilamellar (BLV, 2 concentric vesicles),
- oligolamellar (OLV, liposome with up to 3 concentric vesicles),
- multilamellar (MLV, liposome with more than 3 concentric vesicles),
- multivesicular (MVV, liposome with multiple nonconcentric lipid bilayers) and
- multilamellar-multivesicular (MLV-MVV, liposome with more than 3 concentric vesicles and multiple nonconcentric lipid bilayers).

A liposome that is both multilamellar and multivesicular is considered an MLV-MVV, and neither an MLV nor an MVV. Particularly preferred is an MLV-MVV.

In a preferred embodiment, the phospholipid component consists of DMPC, the liposome comprises a sterol component which consists of cholesterol, the weight ratio of the phospholipid component to the sterol component is 80:20, and the CBD:lipid ratio is 0.42. It is particularly preferred that such a liposome is an MLV-MVV.

In another preferred embodiment, the phospholipid component consists of DMPC, the liposome does not comprise a sterol component, and the CBD:lipid ratio is 0.42. It is particularly preferred that such a liposome is an MLV-MVV.

In yet another preferred embodiment, the phospholipid component consists of DMPC, the liposome comprises a sterol component which consists of cholesterol, the weight ratio of the phospholipid component to the sterol component is 80:20 and the CBD:lipid ratio is 0.7. It is particularly preferred that such a liposome is an MLV-MVV.

In the most preferred embodiment, the phospholipid component consists of DMPC, the liposome does not comprise a sterol component, and the CBD:lipid ratio is 0.7. It is particularly preferred that such a liposome is an MLV-MVV.

During the experimental work which led to the present invention it was also discovered that CBD bioavailability could not be predicted on the basis of the prior knowledge.

It is one of the findings underlying the present invention that in a mixture of CBD-comprising liposomes, CBD bioavailability is increased if there is a larger proportion of multilamellar-multivesicular liposomes (MLV-MVV), multivesicular liposomes (MVV) and/or multilamellar liposomes (MLV), or conversely if there is a smaller proportion of unilamellar liposomes. Unexpectedly, and contrary to the conventional approach, it has also been proven advantageous if the hydrodynamic diameter of CBD-comprising liposomes or the polydispersity index (PDI) is greater than a certain respective threshold, or both.

Accordingly, in a second aspect of the present invention the above object is solved by a mixture of liposomes, wherein the liposomes comprise cannabidiol (CBD), a phospholipid component and optionally a sterol component, and wherein the mixture fulfils one of the following conditions:
a) the proportion by number of multilamellar-multivesicular liposomes (MLV-MVV) in the mixture is at least 10%,
b) the proportion by number of multivesicular liposomes (MVV) in the mixture is at least 5%,
c) the proportion by number of unilamellar liposomes (ULV) in the mixture is at most 70%,
d) the Z-average hydrodynamic diameter, assayed by dynamic light scattering, is greater than 100 nm,
e) the polydispersity index (PDI), assayed by dynamic light scattering, is greater than 0.15,
f) a combination of the above a) and b),
g) a combination of the above a) and c),
h) a combination of the above b) and c),
i) a combination of the above a), b) and c),
j) a combination of the above a) and d),
k) a combination of the above a) and e),
l) a combination of the above a), d) and e),
m) a combination of the above d) and e), or
n) a combination of the above a), b), c), d) and e).

As to the above feature a), the proportion by number of multilamellar-multivesicular liposomes (MLV-MVV) in the mixture is preferably (in the order of increasing preference) 15%, 20%, 25%, 30%, 35%, 40%, 45%, 50%, 55%, 60%, 65%, 70%, 75%, 80%, 85% or 90%.

As to the above feature b), the proportion by number of multivesicular liposomes (MVV) in the mixture is preferably (in the order of increasing preference) 6%, 7%, 8%, 9%, 10%, 11%, 12%, 13%, 14%, 15% or 16%,

As to the above feature c), the proportion by number of unilamellar liposomes (ULV) in the mixture is preferably (in the order of increasing preference) 65%, 60%, 55%, 50%, 45%, 40%, 35%, 30%, 25% or 20%.

In comparison to conventional liposome preparations, it is thus advantageous for oral CBD bioavailability if the mixture comprises more MLV-MVVs, more MVVs and/or less ULVs.

As to the above feature d), the Z-average hydrodynamic diameter is preferably 150 nm, 200 nm, 250 nm, 300 nm, 350 nm, 400 nm, 450 nm, 500 nm, 600 nm, 700 nm, 800 nm or 900 nm, and in particular (in the order of increasing preference) greater than 100 nm and less than 1000 nm, greater than 150 nm and less than 1000 nm, greater than 150 nm and less than 900 nm, greater than 200 nm and less than 800 nm, greater than 250 nm and less than 800 nm, greater than 300 nm and less than 700 nm, greater than 350 nm and less than 700 nm, greater than 400 nm and less than 600 nm, or greater than 450 nm and less than 600 nm.

As to the above feature e) the polydispersity index (PDI) is preferably 0.2, 0.25, 0.3, 0.35, 0.4, 0.45, 0.5, 0.6, 0.7, 0.8 or 0.9, and in particular (in the order of increasing preference) greater than 0.15 and less than 1, greater than 0.2 and less than 1, greater than 0.25 and less than 1, greater than 0.3 and less than 1, greater than 0.3 and less than 0.9, greater than 0.35 and less than 0.9, greater than 0.4 and less than 0.9, greater than 0.4 and less than 0.8, or greater than 0.45 and less than 0.8.

For analysis regarding features d) and e), preferably a Zeta Sizer Nano ZS 90 (Malvern) is used, with which polydispersity index (PDI) and Z-average (harmonic intensity averaged) particle diameter can be measured

Particularly preferred is a combination of a polydispersity index (PDI) of greater than 0.45 and less than 0.8, a hydrodynamic diameter of greater than 450 nm and less than 600 nm, and a proportion by number of multilamellar-multivesicular liposomes (MLV-MVV) of greater than 30%.

In a preferred mixture according to the invention, the liposomes fulfil one or more of the following conditions:
a) the phospholipid component comprises or consists of phosphatidylcholine, wherein the phosphatidylcholine is preferably 1,2-dimyristoyl-sn-glycero-3-phosphocholine (DMPC),
b) the liposomes comprise a sterol component which comprises or consists of cholesterol,
c) the liposomes do not comprise a sterol component,
d) the ratio of the weight of CBD to the total weight of the phospholipid component and, if present, the sterol component (CBD:lipid ratio) is 0.25, 0.42, 0.5, 0.7, 0.8, 1.0, 1.3, from 0.1 to 9, from 0.1 to 4, from 0.1 to 2.3, from 0.1 to 1.3, from 0.25 to 1.3, from 0.42 to 1.0 or from 0.5 to 0.8, wherein the CBD:lipid ratio is preferably 0.7,
e) the liposomes are substantially free from tetrahydrocannabinol,
f) a combination of the above a) and b),
g) a combination of the above a) and b), wherein the weight ratio of the phospholipid component to the sterol component is preferably from 70:30 to 90:10, more preferably from 75:25 to 85:15 and most preferably 80:20,
h) a combination of the above a) and c),
i) a combination of the above a), b) and d),
j) a combination of the above a), c) and d),
k) a combination of the above a), b), d) and e), or
l) a combination of the above a), c), d) and e).

Preferably, the mixture according to the invention has a bioavailability of at least 25%, when assayed in Sprague-Dawley rats at a concentration of 40 mg CBD per kg of body weight in peroral administration, i.e. if administered orally.

A preferred mixture according to the invention has (in the order of increasing preference) a bioavailability of at least 30%, 35%, 40%, 45%, 50%, 55%, 60%, 65%, 70%, 75% or 80% when assayed under the above conditions.

Preferably, the mixture according to the invention has not been subjected to spray-drying.

In an alternative embodiment, the mixture is dried. For example, the mixture may be spray-dried. Spray-drying is preferably performed with isomalt as a carrier, in particular isomalt 720 (available as isomalt GalenIQ^{™} 720 from BENEO-Palatinit GmbH, Mannheim). The dispersing agent is preferably 5% sucrose in water. Preferably, a CBD concentration of sprayed dispersion of 60 to 70 mg/ml (in particular 63.76 mg/ml), a lipid concentration of sprayed dispersion of 85 to 95 mg/ml (in particular 91.00 mg/ml), an amount of 80 to 90 mg CBD per gram of carrier (in particular 85.06 mg/g) and/or an amount of 115 to 125 mg total weight of the phospholipid component and, if present, the sterol component per gram of carrier (in particular 121.4 mg/g) are used (in particular, a combination of these parameters is used). However, a preferred mixture has been dried under mild conditions, such as by freeze-drying. Thus, a preferred mixture is freeze-dried. Freeze-drying is preferably performed with maltose as a carrier. The mixture may be sprayed onto the maltose carrier before freeze-drying.

Yet, it is preferred not to perform spray-drying and more preferred not to perform drying at all. Thus, a particularly preferred mixture has not been subjected to drying.

In a third embodiment the present invention relates to a process for manufacturing liposomes comprising cannabidiol (CBD), comprising:
a) providing CBD, a phospholipid component and optionally a sterol component,
b) providing beads, preferably ceramic beads, more preferably ceramic beads made of zirconium oxide / yttrium stabilised,
c) providing a liquid, preferably selected from the group consisting of water and 5% sucrose in water,
d) providing a vessel with a combination of the beads, the liquid, the CBD, the phospholipid component and, if present, the sterol component,
e) a first step of centrifuging the vessel with the combination in a centrifuge with a rotor which rotates the vessel around two different axes,
f) optionally adding a first additional volume of the liquid to the vessel,
g) a second step of centrifuging the vessel in the centrifuge with the rotor,
h) optionally adding a second additional volume of the liquid to the vessel.

This process allows the production of a liposome or mixture with the above-mentioned advantageous properties.

The optional step f) of adding a first additional volume of the liquid to the vessel after the first step of centrifuging may serve to transfer a vesicular phospholipid gel (VPG, semisolid) into a conventional liposome dispersion by dilution.

Preferably, the process according to the third embodiment of the invention fulfils one or more of the following conditions:
a) the beads have a diameter of 0.8 mm,
b) the beads have a diameter of 1.4 to 1.6 mm,
c) the vessel has an internal volume of 1.5 to 50 ml, preferably 2 to 10 ml,
d) the vessel has an internal volume of 2 ml and is preferably a 2 ml vial,
e) the vessel has an internal volume of 10 ml and is preferably a 10 ml injection flask,
f) the rotor has a first rotation unit and second rotation units, and the vessel is preferably contacted with a second rotation unit such that it is oriented horizontally in relation to the plane of the second rotation unit and/or such that a short axis of the vial is in the same orientation as the axis of rotation of the second rotation unit,
g) a combination of the above a) and e),
h) a combination of the above b) and d)

Preferably, the centrifuge is a Hettich ZentriMix 380 R, and the rotor is preferably the "S-Rotor", both of which are manufactured by Andreas Hettich GmbH & Co. KG, Tuttlingen, Germany. The first step of centrifuging is preferably performed for 15 to 45 min at 2000 to 2700 rpm and 18 to 22°C, in particular 30 min at 2350 rpm and 20°C. The second step of centrifuging is preferably performed for 1 to 3 min at 1200 to 1800 rpm and 18 to 22°C,.in particular 2 min at 1500 rpm and 20°C.

In a very preferred embodiment, the centrifuge is a Hettich ZentriMix 380 R, the rotor is the "S-Rotor", the first step of centrifuging is performed for 30 min at 2350 rpm and 20°C, the second step of centrifuging is performed for 2 min at 1500 rpm and 20°C, the beads are ceramic beads made of zirconium oxide / yttrium stabilised and have a diameter of 0.8 mm, the vessel has an internal volume of 10 ml and is preferably a 10 ml injection flask, and the liquid is 5% sucrose (saccharose) in water. It is particularly preferred to combine this embodiment with a phospholipid component that consists of DMPC, the absence of a sterol component, and a CBD:lipid ratio of 0.7.

According to a fourth embodiment, the present invention relates to a process for manufacturing liposomes comprising cannabidiol (CBD), comprising:
a) providing CBD, a phospholipid component and optionally a sterol component,
b) providing beads, preferably ceramic beads, more preferably ceramic beads made of zirconium oxide / yttrium stabilised,
c) providing a liquid, preferably selected from the group consisting of water and 5% sucrose in water,
d) an optional preliminary homogenising step of the room temperature liquid together with the phospholipid component and, if present, the sterol component in a disperser,
e) a homogenising step of a combination of the liquid, the CBD, the phospholipid component and, if present, the sterol component, and
f) milling the homogenised combination together with the beads in a bead mill having a grinding chamber.

Also this process allows the production of a liposome or mixture with the above-mentioned advantageous properties.

A preferred process according to the fourth embodiment of the present invention fulfils one or more of the following conditions:
a) the beads have a diameter of 0.3 to 0.8 mm, preferably 0.5 to 0.8 mm, more preferably 0.5 mm,
b) the total milling time is 300 min or greater, in particular 330 min,
c) the grinding chamber has been filled with the beads to 50% or greater of its working volume, preferably to 75%,
d) the volume of the combination is 600 to 1200 ml,
e) the maximum agitator speed is 2100 to 2500 rpm, in particular 2150 rpm,
f) the pump speed is 160 to 200 rpm, in particular 165 rpm,
g) a combination of the above a) and b), or
h) a combination of the above a) to e).

Preferably, the bead mill is a Netzsch DeltaVita^{®} 300 Z (NETZSCH-Feinmahltechnik GmbH, Selb, Germany, NETZSCH Vakumix GmbH, Weyhe-Dreye, Germany).

In a particularly preferred embodiment, the bead mill is a Netzsch DeltaVita^{®} 300 Z, the beads are ceramic beads made of zirconium oxide / yttrium stabilised, and the liquid is 5% sucrose (saccharose) in water.

Using the teachings of the present invention, bioavailability may be increased and/or dissolution and permeation properties enhanced as compared to CBD as such, or as compared to marketed liquid formulations.

The present invention also relates to liposomes manufactured by a process of the invention, in particular in one of the preferred embodiments thereof.

Further, the present invention pertains to the liposome according to the invention for use as a medicament, the mixture according to the invention for use as a medicament or the liposomes manufactured by a process of the invention for use as a medicament.

Additionally, the present invention pertains to the liposome according to the invention for use in the treatment of a condition selected from the group consisting of a sleeping disorder, anxiety, depression, a pain disorder, a neurological disorder and spasticity. It also pertains to the mixture according to the invention for use in the treatment of a condition selected from the group consisting of a sleeping disorder, anxiety, depression, a pain disorder, a neurological disorder and spasticity. Additionally, it pertains the liposomes manufactured by a process of the invention for use in the treatment of a condition selected from the group consisting of a sleeping disorder, anxiety, depression, a pain disorder, a neurological disorder and spasticity.

In these uses, the liposome, mixture or the liposomes are preferably administered orally. Additionally mentioned is an oromucosal, a lingual, a sublingual, a buccal, a rectal, a topical or a parenteral administration.

The neurological disorder is preferably selected from the group consisting of epilepsy, Parkinson's disease, multiple sclerosis and Alzheimer's disease.

The present invention also pertains to a pharmaceutical composition comprising the liposome according to the invention, the mixture according to the invention or the liposomes manufactured by a process of the invention, and a pharmaceutically acceptable excipient.

Additionally, the present invention pertains to a capsule, liquid, gel, tablet or stick pack comprising the liposome according to the invention, the mixture according to the invention, the liposomes manufactured by a process of the invention or the pharmaceutical composition according to the invention.

### Examples

Nanoliposomes of CBD with DM PC with and without cholesterol and CBD loadings of 10 to 130% could be made on a lab scale with a dual centrifuge. Initial scale-up experiments were performed on a Netzsch Nanomill. The liposomes were stabilized with sucrose (saccharose) and layered onto isomalt starter particles in a MiniGlatt fluid bed process on a 10 g scale. The first scale up to layer a larger amount had process issues. The granules from two batches were tested in a first rat study vs CBD administered i.v. and the marketed reference product (Epidyolex). Epidyolex from GW Pharmaceuticals is characterised in its Summary of Product Characteristics to contain, per ml of oral solution, 100 mg cannabidiol and as excipients with known effect 79 mg anhydrous ethanol, 736 mg refined sesame oil and 0.0003 mg benzyl alcohol. One prototype showed almost equal bioavailability (22%) compared to the commercial Epidyolex (20%). However, more favourable Tmax (0.5 - 1.8 h) than the commercial product (6.6 h) could be obtained. Liposomal formulations were further optimized in different CBD concentrations 25%, 42%, 50% - with cholesterol and 42%, 70%, 80%, 100% - without cholesterol, respectively, and analysed via cryo-TEM. A selection of five liposomal formulations in different CBD concentrations 42%, 70% - with cholesterol, and 42%, 70%, 130% - without cholesterol were further tested in a second rat study, respectively. CBD i.v and the marketed reference product (Epidyolex) were also administered, respectively. The results showed overall notably improved bioavailability of the liposomal formulation (up to 82.9%). Drying/layering has not been conducted in that series.

The components used are shown in Table 1.

### Example 1: Liposome Preparation Methods

For the purposes of Examples 2 to 6, the following liposome preparation methods were used:

### 2 mL Scale

For a batch size of 300 mg, the lipid and CBD (sum of 50 mg) was weighed into a 2 mL vial (Micro-screw tube from Sarstedt). Details about the lipid (in particular the phospholipid component and, if any, the sterol component, as well as their relative amounts, if applicable) in the individual preparations are given in Tables 2 to 7. 600 mg silibeads Zy-S 1.4-1.6 mm (ceramic yttrium stabilised zirconium oxide beads, Sigmund Lindner, Warmensteinach, Germany) and 50 µl dispersing agent was added. Details about the dispersing agent in the respective preparations are also given in Tables 2 to 7. All previous steps were performed at room temperature.

Dual centrifugation with a centrifuge ZentriMix 380 R, S-rotor (Hettich, Tuttlingen, Germany) was performed. The vials were horizontally oriented in relation to the plane of the second rotation unit and 40°C tilted in relation to the primary rotation unit. In the first step, the following parameters were set: 2350 rpm, 30 min at 20°C. The mixture was diluted with 200 µl dispersing agent and centrifuged at 1500 rpm for 2 min at 20°C.

For analysis, 3 µl dispersion was diluted with 3 ml dispersing agent and further analysed with the Zetasizer Nano ZS 90 (Malvern) and the Z-average hydrodynamic diameter r and the polydispersity index (PDI) was determined.

### 10 mL Scale

For a batch size of 6 g, the lipid and CBD (sum of 1 g) was weighed into a 10 mL injection flask (PP-flask from Iphase). Details about the lipid (in particular the phospholipid component and, if any, the sterol component, as well as their relative amounts, if applicable) in the individual preparations are given in Tables 2 to 7. 3.5 g silibeads Zy-S 0.8 mm (ceramic yttrium stabilised zirconium oxide beads, Sigmund Lindner, Warmensteinach, Germany) and 1 mL dispersing agent was added. Details about the dispersing agent in the respective preparations are also given in Tables 2 to 7. All previous steps were performed at room temperature.

Dual centrifugation with a centrifuge ZentriMix 380 R, S-rotor (Hettich, Tuttlingen, Germany) was performed. The vials were horizontally oriented in relation to the plane of the second rotation unit and 40°C tilted in relation to the primary rotation unit. In the first step, the following parameters were set: 2350 rpm, 30 min at 20°C. The mixture was diluted with 4 ml dispersing agent and centrifuged at 1500 rpm for 2 min at 20°C.

For analysis, 3 µl dispersion was diluted with 3 ml dispersing agent and further analysed with the Zetasizer Nano ZS 90 (Malvern) and the Z-average hydrodynamic diameter and the polydispersity index (PDI) was determined.

### Scale-up : 600 mL - 1200 mL

To perform experiments with lipid compositions and agitator bead mill Netzsch DeltaVita^{®} 300 Z volumes of 600 - 1200 mL were used. The preparation of each batch is described in Example 4 under the heading "Liposome formulation using a bead mill (Netzsch Nanomill)". The grinding chamber of the agitator bead mill was filled to 75% of the working volume with ceramic beads.

### Example 2: Liposome formulation (lab scale)

### Liposome formulation by dual centrifugation

In a first screening, three different lipids, S100, 90-H and DMPC, with various loads of CBD (from 9.9%, 30%, 50%, to 70%) were tested (see Table 2). The screening was performed in a batch size of 2 mL. The liposomes were prepared according to Example 1. For evaluation of the liposomal dispersion, hydrodynamic diameter and the PDI was used, both of which were measured by dynamic light scattering (DLS).

Conventionally, for liposomes, PDI values ≤ 0.3 are considered acceptable, which indicate homogenous dispersions. A hydrodynamic diameter ≤ 300 nm indicates unilamellar liposomes. Thus, the following evaluation of particle size was used, which uses such conventional standards (low PDI values, low hydrodynamic diameter) and allocates the parameters to certain groups, which are herein called A, B and C:

| **Group** | **Properties** |
|---|---|
| **A** | Hydrodynamic diameter ≤ 300 nm, PDI ≤ 0.3 |
| **B** | Hydrodynamic diameter ≤ 300 nm, PDI ≤ 0.5 |
| **C** | Hydrodynamic diameter ≥ 300 nm, PDI ≥ 0.5 |

The allocation of liposomes to groups A, B and C is indicated in Tables 2 to 10, 12 and 24.

The most promising candidate out of the screening (Li001-Li012) showed the lipid DMPC with a CBD load of 10%. In the next trial, the CBD load was altered in 5% increments from 10 to 30% (Li013-017). Addition of 20% cholesterol to the lipid phase did not increase the possible CBD load (Li018-022). A scale-up with a total batch size of 6 mL was performed with the formulation of Li013 and Li014 (Li023-025). These batches were used for analytical tests (Li023-024; Li025-029) and for a first spraying test (Li025). As liposomes did not stay intact after spraying onto isomalt 720, drying and resuspending in the dispersing agent two different stabilizing agents were tested: 5% mannitol (Li030-031) and 5% saccharose (Li032-061; Li066-113). These batches were further used for spraying trials. A side batch with the aim to produce no intact liposomes was performed in Li114, an overload of 70% CBD was used. Larger batch sizes of the side batch were produced in Li115-122. Another side batch without the stabilizing agent saccharose was produced in Li123-126. Batch Li127-142 was used with the stabilizing agent saccharose and 70% CBD overload and the following granules were used for stability studies. The liposomal dispersion of Li143-158 and the following granules were used for stability studies.

Further liposomal dispersions Li163-178 and Li179-195 were reproduced according to batches Li127-142 and Li143-158, respectively. Their corresponding granules from batches C020 and C021, respectively, were used in the rat study. Liposomal dispersions Li196-203 and Li204-211 were reproduced as fresh batches for the side batch and prototype batch, respectively, and further analysed via cryogenic transmission electron microscopy (cryo-TEM).

The scope is to obtain high CBD bioavailability, regardless of the formulation. The following section will include results from different strategies into optimizing CBD:lipid ratio and the lipid content, as liposomal dispersion.

### Optimisation of CBD:lipid ratio and lipid type (after the first rat study)

The first animal study (Example 5) revealed that the side-batch achieved a 22% bioavailability. This result slightly exceeded the one in the commercial product, Epidyolex. To improve the bioavailability further, additional optimizations of the liposomal formulation were followed upon. As depicted in Table 5, CBD was mixed in different ratios with different lipid mixtures with the purpose to achieve a higher CBD concentration and an increased number of multilamellar liposomes.

The lipid mixtures and CBD:lipid ratios that were initially investigated were as follows: for the DMPC lipid CBD:lipid ratios of 9, 4, 2.3, 1.5, 1.0, 0.66, 0.42, 0.25, 0.11 (229717Li207-215) were screened with the zetasizer and compared to the reference batch (229717Li206), which contains 0.7 CBD:lipid ratio. The CBD:lipid ratios that showed comparable analytical values were 0.66, 1.0, 1.5.

The next lipid mixture investigated was containing 80% DMPC and 20% cholesterol (Chol). Forthis, CBD:lipid ratios of 9, 4, 2.3, 1.5, 1.0, 0.66, 0.42, 0.25, 0.11 (229717Li216-224) were screened with the zetasizer and compared to aforementioned reference batch (229717Li206). Subsequently, the ratio intervals that compared the most with the reference batch was 0.25-1.0. Smaller concentration increments from this range were followed upon.

The last lipid mixture investigated was one containing 90% DMPC and 10% Chol. For this, CBD:lipid ratios of 9, 4, 2.3, 1.5, 1.0, 0.66, 0.42, 0.25, 0.11 (229717Li225-233) were screened with the zetasizer and compared to the aforementioned reference batch (229717Li206). In this case, the ratio intervals that compared the most with the reference batch was 0.42-1.0. Further optimizations were not performed for this batch.

General observations that were made based on these results:
- the more CBD (ratio 9-1.5) the more polydisperse the liposomes
- CBD:lipid ratios between 1 - 0.42 yields generally more homogeneous liposomes, suitable for further optimizations.

To find the highest CBD:lipid ratio with DMPC alone that compares to the reference batch, 0.1 concentration increments in the 0.66 - 1.5 interval were followed upon in batches Li234-243. Similarly, 80% DMPC with 20% cholesterol as the alternative lipid source within the 0.66-1.0 CBD:lipid ratio interval were performed in batches Li244-248 to find the highest CBD:lipid ratio that compares to the reference batch. An additional, complementary qualitative method was desired to analyse the data. Cryogenic Transmission Electron Microscopy (cryo-TEM) was the method of choice, since it is a precise and direct method to determine liposome lamellarity, size, shape and ultrastructure.

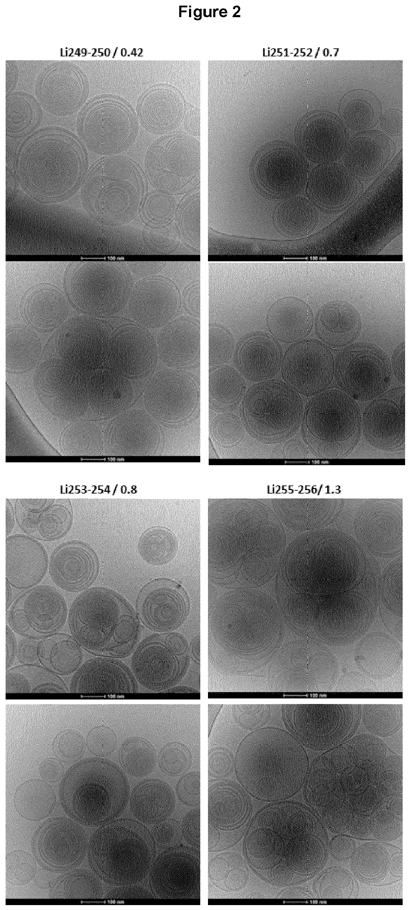

Thus, a selection of liposomes were further analysed with cryo-TEM (Figure 2) Liposomal dispersions with DMPC were analysed for CBD:lipid ratio of 0.42 (Li249-250), 0.7 (Li251-252), 0.8 (Li253-254) and 1.3 (Li255-256). Liposomal dispersions with 80% DMPC and 20% cholesterol were also analysed, with CBD:lipid ratios of 0.25 (Li257-258), 0.42 (Li259-260), 0.5 (Li261-262) and 1.0 (Li263-264).

For the analysis of the micrographs, multilamellar liposomes (MLV) and in particular multilamellar-multivesicular liposomes (MLV-MVV) were considered particularly important with respect to a higher degree of encapsulation efficiency. Thus, according to Figure 2, all the liposomal dispersions with only DMPC as a lipid source contain multilamellar or multilamellar-multivesicular liposomes. An exception is batch Li255-256 /1.3, where liposomes appear to be heterogeneous in size and structure. The difference might lie in the fact that a higher CBD:lipid ratio was used than in the other 3 batches.

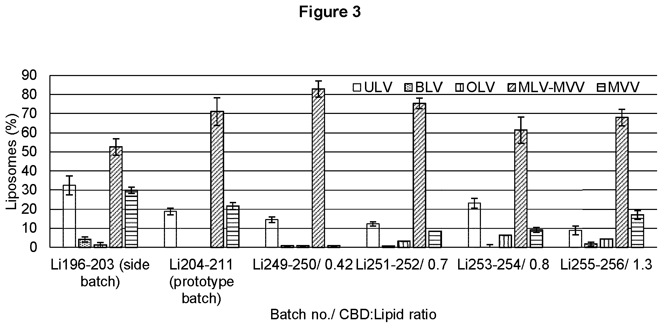

For a better understanding and analysis of the micrographs, the batches were further analysed by the quantification of the liposome types: unilamellar (ULV), bilamellar (BLV), oligolamellar (OLV), multilamellar-multivesicular (MLV-MVV) and multivesicular (MVV) as described later in Example 5, Part (v). The results are depicted in Figure 3.

The following observations were made based on the quantification. Some variability in the amount and types of liposomes is observed between batches Li251-252 /0.7 and Li196-203 (side batch), in which the same CBD:lipid ratio was used (0.7). For example, an amount of 75% of MLV-MVV is quantified in batch Li251-252 /0.7 and 52% MLV-MVV in batch Li196-203 (side batch). Also, the amount of ULV is higher in batch Li196-203 (32%), compared to batch Li251-252/ 0.7 (12%). This could be due to slight differences in handling (in lab or grid preparation).

Furthermore, the highest no. of MLV-MVV is observed in 0.42 CBD:lipid ratio (83%), followed by 0.7 (75%) and lastly by 0.8 (61%). The 1.3 CBD:lipid ratio displayed the most heterogeneous MLV-MVV liposomes (69%).

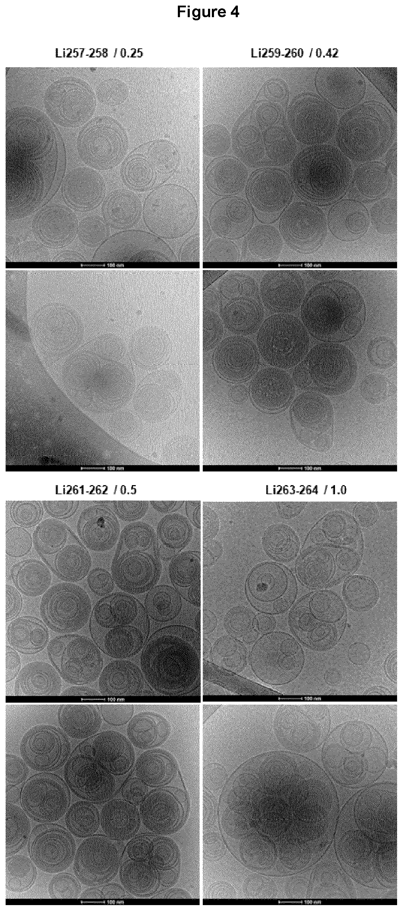

Next, the effect of cholesterol was investigated on the liposome lamellarity. For this, CBD: lipids (80% DMPC and 20% cholesterol) in a ratio of 0.25 (Li257-258), 0.42 (Li259-260), 0.5 (Li261-262) and 1.0 (Li263-264), respectively, were evaluated via cryo-TEM, respectively (Figure 4)

According to Figure 4, all the liposomal dispersions in which DMPC and cholesterol were used as a lipid source contain multilamellar liposomes in terms of size and multilamellarity. Batches Li261-262/0.5 and Li263-264 /1.3 are the exception, where liposomes appear to be heterogeneous. The reason for that could have been the higher CBD:lipid ratio that was used compared to the other two batches. Cholesterol might have exerted a stabilizing effect on the liposomes, possibly by rearranging itself between the hydrophobic moieties of the lipids, which might also have interfered with the liposome formation.

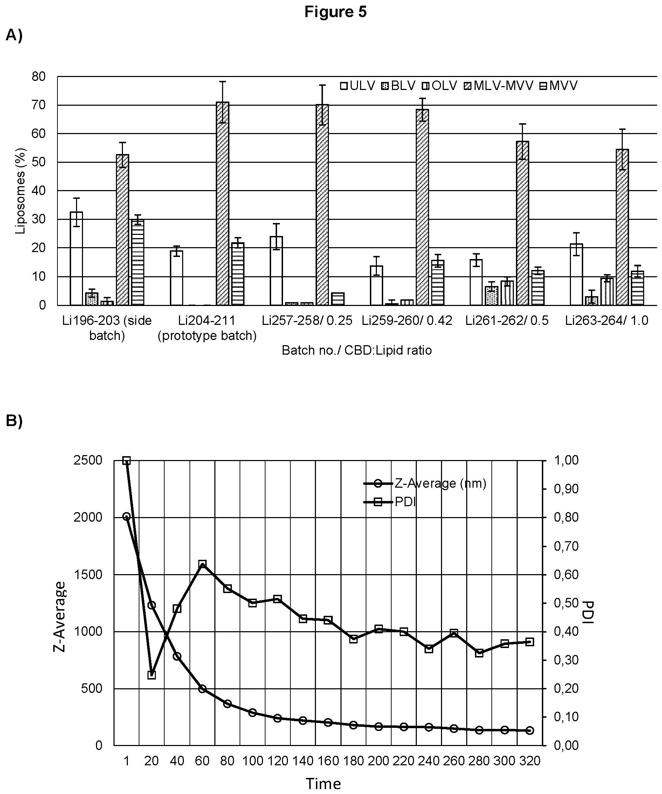

For a better understanding and analysis of the micrographs, the batches were further analysed by the quantification of the liposome types. The results are depicted Figure 5.

The following observations were made based on the quantification. MLV-MVV no. are comparable between 0.25 (79%) and 0.42 CBD:lipid ratio (68%). 0.5 and 1.0 ratios yielded comparable amounts of MLV-MVV, 57% and 54%, respectively. This might be explained by the fact that cholesterol has a similar structure to CBD. This could determine a competition effect between the two for membrane insertion. This is also supported by the encapsulation efficiency results (EE%). As listed in Table5, the encapsulation efficiency is lower in the samples with cholesterol (61%-81%), than without cholesterol (78%-93%). Furthermore, the EE% decreases when more CBD is used.

### Conclusion and formulation for the second rat study

In order to test the optimized formulations mentioned in the previous section ("Optimisation of CBD:lipid ratio and lipid type (after the first rat study)"), a second rat study was planned. A few changes in the CBD:lipid ratios were made, for an easier comparison between the formulations.

Five formulations were produced for this purpose. Three batches with cholesterol were produced with CBD:lipid ratios of 0.42 (Li297-298), 0.7 (Li299-300) and 1.3 (Li301-302), respectively, and two batches without cholesterol with CBD:lipid ratios of 0.42 (Li303-304), and 0.7 (Li305-306) respectively (Table 5).

### Example 3: Spraying trials

Liposome dispersions prepared according to Example 1 were sprayed on the solid carrier Isomalt 720 in order to obtain solid liposomal formulations. Spraying trials were performed with the fluidizer bed system Mini-Glatt using top spray method and a Micro Kit which is suitable for a volume of 5-100 mL. The dye with an opening of 0.3 mm and cap A was used.

In the first trial, the liposomal dispersion 229717Li025 was used. Stability of the liposomes was determined by measuring the hydrodynamic diameter of the dispersion after 5 days. The liposome dispersion was diluted to a lipid concentration of 90.97 mg/mL and a CBD concentration of 9.00 mg/mL. The dispersion was layered onto 10 g Isomalt 720. In 229717C001, a final amount of 82.78 mg lipid per g carrier relating to a CBD amount of 8.19 mg per g carrier was sprayed. After redispersion the hydrodynamic diameter and PDI was measured. After spraying and drying the liposomes did not stay intact, which is the reason why in the next trials stabilizers were added to the aqueous phase.

In 229717C002 5% mannitol was added to ddH₂O. A final amount of 113.94 mg lipid per g carrier relating to a CBD amount of 11.27 mg per g carrier was sprayed. Redispersion did not show intact liposomes.

The addition of 5% saccharose and a final concentration of 13.05 mg CBD/g carrier and 136.55 mg lipid/g carrier led, after redispersion, to intact liposomes with a hydrodynamic diameter of 170 nm (C003).

The undiluted liposome dispersion with a concentration of 15 mg CBD/ml and 151.67 mg lipid/ml and a final layered CBD amount of 15.75 mg/g carrier and 159.25 mg lipid/g carrier did not lead to intact liposomes after redispersion.

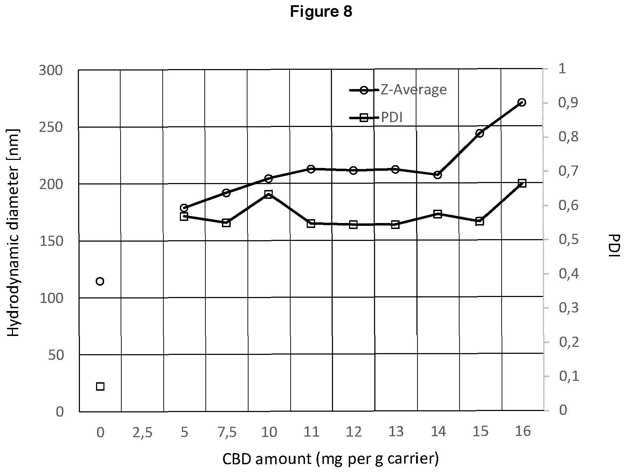

In C005, the liposomal dispersion was diluted like in batch C001-C003 to 9 mg CBD/ml and 91 mg lipid/ml. In C005, 10 samples were drawn after various amounts of layered liposome dispersions. In Figure 8, the hydrodynamic diameter and PDI of the drawn samples with increasing amount of layered liposome dispersion are resumed. A shift of the hydrodynamic diameter is shown between 14-15 mg CBD/g carrier, which indicates the maximal amount of sprayed liposome dispersion before lipid agglomeration appears. In C006, a final layered amount of 10 mg CBD per g carrier was aimed, product temperature was kept at 25°C. After redispersion in 5% saccharose, liposomes had a hydrodynamic diameter of 188.8 nm. A high loss of isomalt at the filter was detected (loss of 61.5%).

In C007 a larger batch size of 40 g isomalt was tested in order to reduce the relative loss of yield, still a high loss of 44% was observed. Placebo tests with isomalt alone in the fluid bed system without spraying led after 45 min to a loss of 50% (filter blowout every 4 s). Direct spraying of ddHzO after starting the process led to a loss of 22% (filter blowout every 4 s). Variation of the filter blowout from 2 s and 8 s resulted in a loss of 26% and 24%, respectively. For the following experiments filters were pre-powdered with isomalt 720 in order to decrease loss of yield.

In the next batch (C008), the influence of the product temperature was tested by increasing the product temperature to 30°C. Hydrodynamic diameter was increased to 237.5 nm and PDI 0.56. For the next spraying trials the product temperature was kept below 30°C, preferably at 25°C.

In order to stabilize liposomes, a 10% saccharose-solution was used in batch Li062-065 used for layering in C009, the process had to be stopped after 36 min (relates to 3.6 mg CBD/g carrier) as the dye was clogged and the isomalt was sticking to the reservoir wall.

Upscaling of the process to 100 g isomalt was performed in C010. Therefore, the Mini Kit was used and the dye 0.8 mm with the cap B. A yield of 62.3% was received.

In the next batch C011 process parameters were optimized in order to increase the yield and to receive intact liposomes after redispersion. Therefore, inlet airflow was decreased to an average of 9.34 m³/h, product temperature was kept ≤ 25°C, spraying pressure and rate was decreased to 0.63 bar and 0.84 g/min. Product was dried for 5 min at a product temperature ≤ 25°C. The yield was increased to 99.5%. LoD was measured for 15 min at 100°C: 6.04%. Additional tray drying at 25°C for 10 min led to a LoD of 6.22%, additional tray drying at 30°C for 2 h led to a LoD of 6.12%.

In the next batch, C012, reproducibility was tested and the process was driven with the same parameters as in C011. After spraying, the product was dried for 10 min in the fluid bed. Additional fluid bed drying with an amount of 20 g was performed for 3 h at a product temperature of 25.3°C. After 1 h LoD was 5.07% and after 3 h 4.92%. Vacuum drying for 6 h at 23°C and a pressure of 60 mbar yielded an LoD of 5.52%. A significant decrease of moisture could not be reached with the tested methods.

Due to a process issue (breakage of glass) in C013 Li115-118 the process had to be stopped and discarded in advance.

In C014 the experiment of C013 was repeated.

In C015 the liposomal dispersion without the stabilizing agent saccharose was layered onto isomalt.

A lager batch size of C014 was produced in C016. This batch was used for first stability studies.

C017 was a reproduction of C012 and was also used for first stability studies.

Further on, new batches C020 and C021 were similarly reproduced after C016 and C017, respectively. The two new batches were further used in the rat study.

### Example 4: Scale-up of liposome formulation

### Liposome formulation using a bead mill (Netzsch Nanomill)

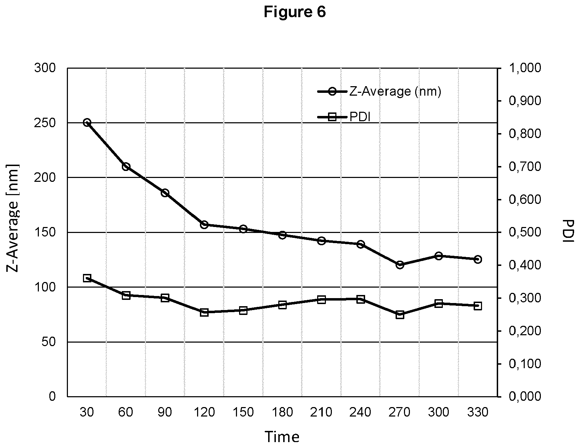

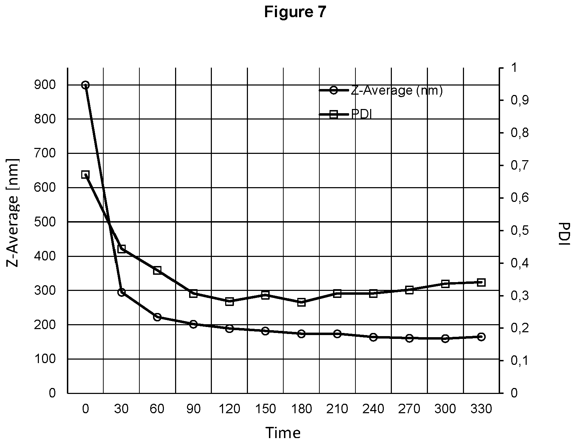

A first upscale batch (229717LN001) was performed with Silibeads Zy-S, size: 0.8 mm (ceramic yttrium stabilised zirconium oxide beads, Sigmund Lindner, Warmensteinach, Germany), see Table 9. All ingredients were weighed into a beaker and homogenized with an ultra-turrax. Start CBD concentration was 15.0 mg/g, the dispersion was too viscous and the pressure too high, which is why the dispersion was diluted with H₂O + 5% saccharose to a final CBD concentration of 12.2 mg/g. Agitator speed was constantly increased to 2350 rpm and pump to 185 rpm. A final hydrodynamic diameter of 132.0 nm and a PDI of 0.364 was reached after 320 min (Figure 5). In the next batch, smaller silibeads of 0.3 mm were used in order to decrease the PDI. Dispersion was to viscous and could not be pumped through the system. Therefore, the process was stopped. In batch 229717LN002, silibeads 0.5 mm were used (see Table 9). The 5% saccharose solution was preheated to room temperature (21°-23° C), the lipid was added and homogenized during the night with a propeller stirrer (IKA EUROSTAR 20 digital), at the speed of 1 000 rpm, room temperature. The next morning, CBD was added and mixed with the ultra-turrax (IKA T25 digital ultra-turrax) at the lowest speed (11 000 rpm) until completely homogenized (20 min) before starting the experiment. The dispersion was added into the beaker of the agitator bead mill. The process progressed without any issues, and parameters are listed in Table 9. Analytic results are displayed in Figure 6. 229717LN003 was performed like 229717LN002. Analytic results of LN003 are displayed in Figure 7 and parameters listed in Table 9.

### Spraying trials

First scale up trials were performed at the fluid bed system Unilab. The first batch of the liposome dispersion from the bead mill (219716LN001) described in in this Example 4 above was divided into two parts. The small product beaker (7.5 I) was used for the Unilab. Filter with a mesh size of 15-25 µm, the 1.2 mm dye, micro-climate at 0.03 bar and spraying 0.3 air at 0.3 bar were used. An amount of 360 g Isomalt 720 was used. Various parameter settings were tested. The process had to be stopped due to aggregation of the production (C018).

In C019 the larger beaker (13.4 I) was used with a loading of 742.5 g. The process could be run until the end, but strong agglomeration of the product was observed. However, a three times bigger amount of loading should be performed in order to increase the flow of the fluid bed.

### Example 5: First rat study

The first rat study, the results of which are included in the general overview of rat studies and characterisation of prototype batches (Table 10), was performed as follows:

### Part (i) - Predevelopment phase

### Solubilisation of CBD granules for oral rat feeding

Granules with CBD concentrations of 1 mg/ml, 2 mg/ml and 4 mg/ml from batch 229717C018 were dissolved in ddH₂O. The resulting dispersions were analysed for sedimentation, hydrodynamic diameter and PDI.

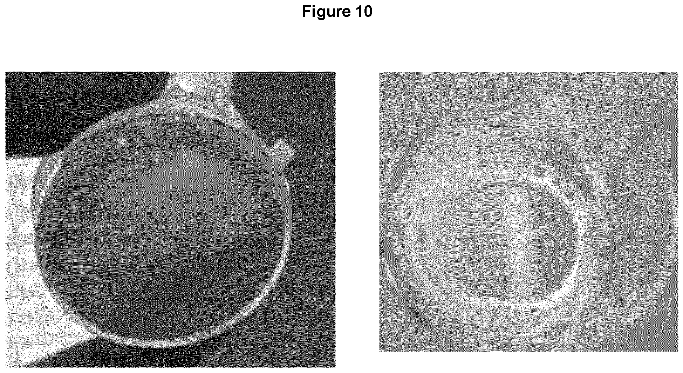

Upon water dispersion, a sediment formation was observed in all of the concentrations tested (Figure 10). The retrieved Z-sizer results had poor quality. This is likely due to particles found in the sediment that interfere with the measurement.

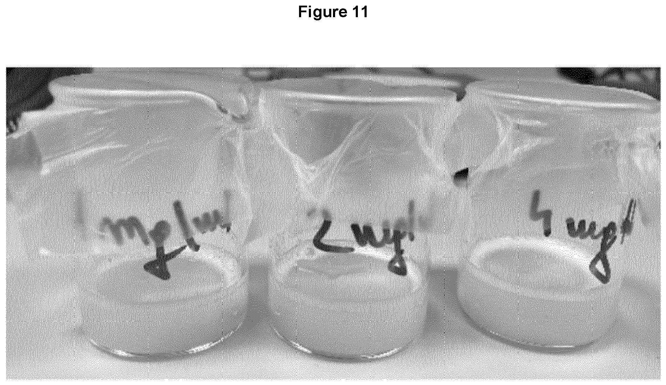

To test whether the sediment is due to talc, batch 229717C017 was further analysed. This batch also contains 12 mg CBD/g carrier and no talc. As expected, no sediment formation and a good Z-sizer quality result were observed (Table 11 and Figure 11).

As displayed in Table 11, batch 229717C017 yielded overall better Z-sizer values than batch 229717C018, despite dispersion settling and filtration. The best results of all the conditions tested were observed in the dispersion containing 4 mg/ml CBD (in bold) from batch 229717C017 (180 d.nm hydrodynamic diameter and 0.326 Pdl). This batch was stored in the dark at room temperature (21°-23° C). The 4 mg/ml CBD concentration was further used in the first rat study.

Considering its liposome stabilizing properties, 5% sucrose (saccharose) was further used as a dispersant for 229717C017 batch. This investigation was performed 13 weeks after its production. The granules contained a theoretical concentration of 12 mg CBD/ g carrier. Granules with CBD concentrations of 1 mg/ml, 2 mg/ml and 4 mg/ml were analysed in 5% sucrose and 4 mg/ml in ddHzO for comparison. The resulting dispersions were analysed for hydrodynamic diameter and PDI (Table 12).

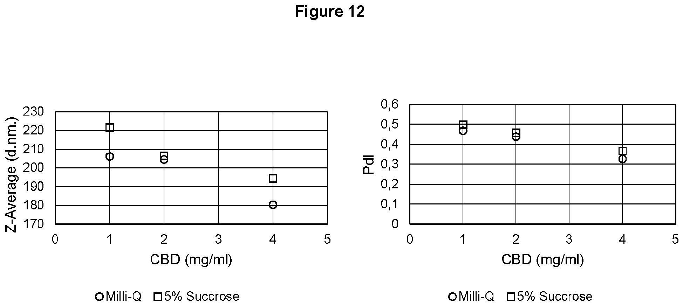

For the 4 mg/ml CBD granules dissolved in ddHzO, <200 d. nm. value for hydrodynamic diameter and PDI under 400 were retrieved (Figure 12). However, the values were slightly increased as compared to the values retrieved 10 weeks after granule production. Moreover, the 4 mg/ml CBD granules dissolved in ddH₂O and in 5% sucrose, respectively, revealed comparable hydrodynamic diameter and PDI, regardless of the solute used. For 1 mg/ml and 2 mg/ml CBD granules dissolved in 5% sucrose, a similar tendency was observed as for the granules dissolved in ddH₂O: the higher the solute concentration, the smaller the hydrodynamic diameter and PDI (Figure 12). This effect might be due to a stabilizing effect of sugars onto liposomes in the rehydration process. Isomalt was the carrier used in the granules production, which is also a sugar, composed of a mixture of glucose, mannitol and sorbitol. This could explain the slight stabilization of liposomes which increases with higher isomalt concentrations.

Next, batches 229717C016 and 229717C017 were reproduced as new batches 229717C020 and 229717C021, respectively, for the first oral rat study. The prototype batch 229717C021 contained a theoretical concentration of 12 mg/g CBD/carrier, whilst the side-batch 229717C020 was included in the study to make up for a higher theoretical CBD concentration of 85 mg/g CBD/carrier. Their respective granules were ddH₂O and 5% sucrose - dispersed, respectively, and their hydrodynamic diameter and CBD encapsulation efficiency were measured (Table 5 and Table 12). For batch C020, out of the range hydrodynamic diameter and PDI values were expected. As a result, dispersion in 5% sucrose yielded a hydrodynamic diameter of 649.3 d.nm., which was lower than the one retrieved in ddH₂O of 831.5 d.nm. The PDI yielded comparable values of 0.952 and 0.950 in ddH₂O and 5% sucrose, respectively. For this batch, 5% sucrose appeared to reconstitute a lower hydrodynamic diameter than ddH₂O, which is conventionally desired. For batch C021, hydrodynamic diameter and PDI were expected to be in range.

Thus, regardless of the dispersant used, corresponding values were fitting into the expected range (Table 1 and Table 5).

Overall, ddHzO yielded hydrodynamic diameter values that fit the expected range, so it was further used as a dispersant over 5% sucrose for simplicity.

The theoretical encapsulation efficiency of CBD in nanoliposomes of C020 and C021 was planned to be 85.0 mg/g carrier and 12 mg/g carrier, respectively. After spraying, the encapsulation efficiency of CBD was determined for C020 to be 61.2 mg/g carrier and for C021 to be 9.18 mg/g carrier. The lower values fit into the expected range of CBD encapsulation efficiency.

Therefore, the amounts for the oral formulations were further adjusted for 4 mg/ml CBD concentration in ddH₂O, and the hydrodynamic diameter values were determined. As a result, the side-batch C020 yielded values of 990.9 d.nm. and a PDI of 0.934, and the prototype batch C021 yielded values of 145.7 d.nm. and 0.264 PDI. The values for the prototype batch were within the expected range, whilst the ones for the side-batch were not. However, these effects were not unexpected, given that the side-batch had a CBD and liposome overload, which could lead to high PDI and hydrodynamic diameter values.

### Preparation of commercial CBD as reference for oral rat feeding

As a control for the CBD granules, a commercial product was used, namely Epidyolex. It contains 100 mg/ml CBD, 10% ethanol (v/v), sesame oil, and strawberry flavour with benzyl alcohol. Sesame oil was further used for diluting the CBD to the concentration that matched the target for the oral rat study 1 mg/ml, 2 mg/ml and 4 mg/ml, respectively. As displayed in Figure 11, each dilution turned out to be visually clear, with no flakes or cloudiness.

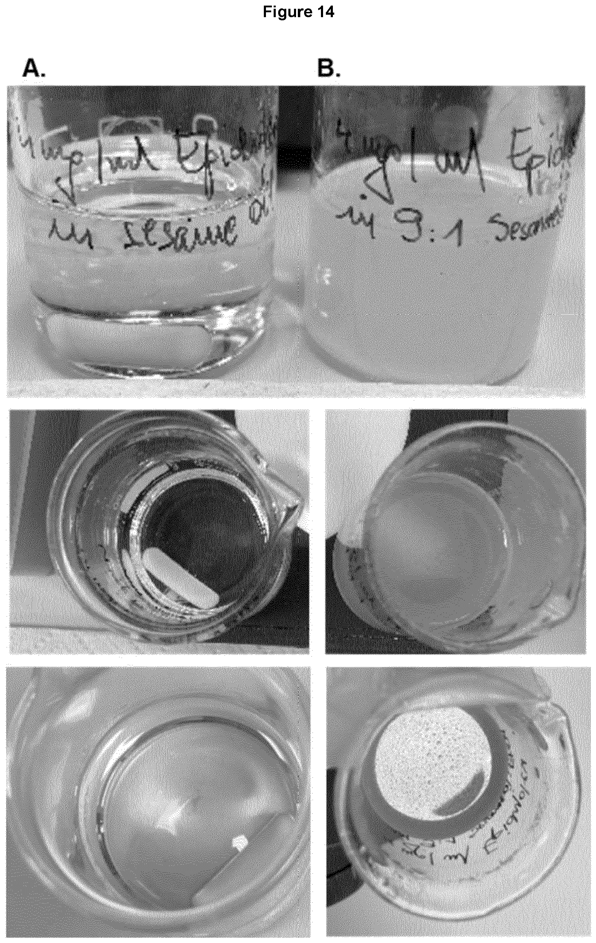

Another combination was also investigated, where 9:1 sesame oil: ethanol absolute was prepared, according to the commercial product (Figure 14B). However, this mix turned out turbid and only led to a clear solution when stirred at 55° C for 5 min. This effect was reversed when cooled down to 32° C; the solution became turbid with superficial droplets (Figure 14B). That could be due to insufficient stirring time.

For simplicity, sesame oil was further used for commercial CBD dilution.

### Preparation of the intravenous formulation of CBD

To obtain an intravenous formulation, two different approaches were first tested.

For the first approach, 50 mg/ml CBD was dissolved in 95% ethanol and further diluted to 1 mg/ml CBD in 96% saline and 2% polysorbate 80. For the second approach, 50 mg/ml CBD was dissolved in 1:1 ethanol: polysorbate 80 and further diluted to 1 mg/ml CBD in 98% saline.

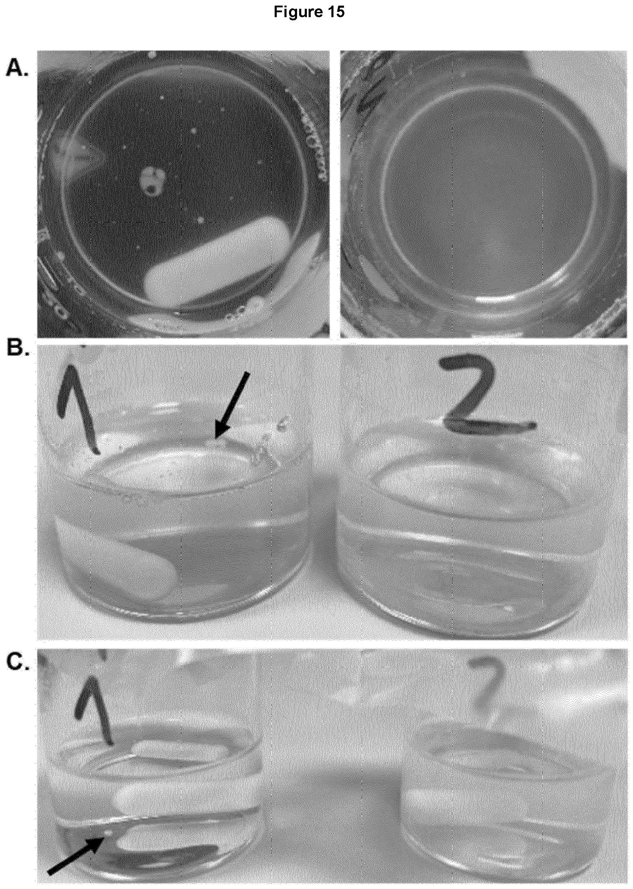

In both situations, 50 mg/ml CBD could be dissolved. However, for the first approach the dilution to 1 mg/ml, led to the formation of fat drops (Figure 15A), which could not be dissolved after 10 min stirring (Figure 15B), nor after 10 min heat stirring (Figure 15C). For the second approach, the formulation looked turbid after diluting (Figure 15A), no fat drops and no sediment were observed after 10 min or 20 min rest at room temperature (Figure 15B and C). The turbid effect could be due to the surfactant effect of polysorbate 80 with CBD and saline solution. When comparing the two solutions, the second approach qualified as a suitable injectable formulation for the rat study.

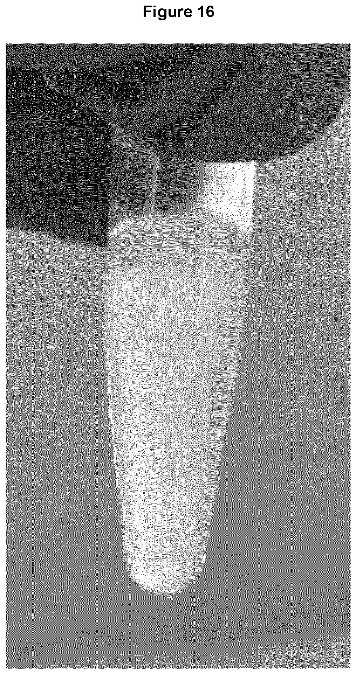

A third approach was attempted, wherein 50 mg/ml CBD was dissolved in ethanol and diluted to 5 mg/ml in sesame oil, reaching a final ratio of 9:1 sesame oil: absolute ethanol. However, as stated in the previous subsection, this mix turned out non-uniform (Figure 16) and only led to a clear solution when stirred at 55° C for 5 min. That again might be due to insufficient stirring time.

According to these results, the second approach involving a tween and saline solution reached a stable and uniform solution. Polysorbate 80 could act as an emulsifier for CBD. Importantly, polysorbate 80 has to be added in the stock solution, as its emulsifying properties are lost when added only in the final diluted solution.

The second approach was further used as the intravenous formulation, given that there was CBD solubility and no sediment formation.

### Conclusion for predevelopment phase- oral and intravenous

For the oral rat study, the concentration in which the CBD nanoliposomes were most stable was 4 mg/ml CBD of granules from batch 229717C017. This concentration was to be further used in the oral rat study.

As a control for the 229717C017 batch used in the oral rat study, the commercial product, Epidyolex, was to be further used in a comparable concentration of 4 mg/ml CBD, diluted in sesame oil.

For the intravenous study, 50 mg/ml CBD dissolved in 1:1 ethanol: Tween 80 and further diluted to 5 mg/ml CBD in 98% saline was the formulation of choice. Considering the volume limitation that can be injected per rat, a dose of 1 mg CBD can be injected from the developed formulation.

### Part (ii) - Development phase - granules

To acquire a first understanding on the pharmaceutical kinetics of the side batch and the prototype batch, a small scale rat study was conducted.

As displayed in Table 10 and in Table 13, granules from the side-batch 229717C020 and the prototype batch 229717C021 were orally administered (F2-F4 arm number), respectively, and results calculated against the intravenously administered commercial formulation (F1 arm number). Additionally, the commercial CBD-containing product Epidyolex was used as a reference, and ddH₂O was used as negative control. Three Sprague-Dawley rats were tested for each study arm. Concentrations and administered amount are displayed in Table 13.

### Part (iii) - Analytic results of the formulations

The granules used in the first rat study were further analysed for their hydrodynamic diameter, PDI, encapsulation efficiency and purity. The values are listed in Table 14. Consequently, the CBD amount, hydrodynamic diameter and the PDI increase with the amount of CBD.

### Part (iv) - Pharmacokinetic results

The formulations prepared prior to administration were milky solutions with no bubble for Formulations 1, 2, 3 and 4 (F1, F2, F3 and F4, respectively, see Table 13). Formulation 5 (F5) was a clear and viscous solution. Table 15 displays the pharmacokinetics parameters.

The formulations were well tolerated by the animals with no clinical signs observed, with the following exception: animal PO13 died around 6 hours after administration of Formulation 5 without showing any preliminary sign of toxicity, and the dissection did not reveal any abnormality.

After IV administration of Formulation 1 (5 mg/kg), the compound was measurable in the samples up to 24 hours and was below the limit of quantification at 48 hours.

After oral administration of Formulation 2 (20 mg/kg, 5 mL/kg), the kinetics profile was reproducible between the animals of a same group. The compound was measurable up to 8 hours in two of three animals and up to 24 hours in one animal.

After oral administration of Formulation 3 (40 mg/kg, 10 mL/kg), the kinetics profile was reproducible between the animals of the same group. The compound was measurable up to 24 hours in all subjects' blood samples and up to 48 hours in one subject.

After oral administration of Formulation 4 (40 mg/kg, 10 mL/kg), the concentrations were measurable up to 24 hours in the blood samples of all subjects.

After oral administration of Formulation 5 (40 mg/kg, 10 mL/kg), the kinetics profile was comparable between two of the three subjects with variability of the maximal concentrations.

For one subject the kinetics was stopped at 6 hours because of unexpected death of the animals.

After IV administration of Formulation 2, the mean extrapolated concentration at T0 was 9351 ng/mL, mean AUCt 5134,2 h*ng/mL and half-life was 6.7 hours for one subject after exclusion of the profiles when elimination phase of the curve was not accurately defined (AUCextra > 20% or Rsq <0.8)

Regarding the absorption of the compound in different formulations, the mean observed Cmax and exposure over time (AUCt) increased proportionally with dose between Formulation 2 (20 mg/Kg) and Formulation 3 (40 mg/kg).

For a same dose (40 mg/kg), the mean of maximal concentrations observed and the mean exposure over time were higher after administration of Formulation 4 in comparison of Formulation 3.

On the contrary, the comparison of Formulation 2 (20 mg/kg) with Formulation 5 (40 mg/kg) showed that concentrations were sub-proportional with the dose.

The observed Tmax was comparable between Formulations 2 (10 mg/kg, 5 mL/kg) and 3 (40 mg/kg, 10 mL/kg) and slightly delayed for Formulation 4 (40 mL/kg, 10 mL/kg).

For Formulation 5 (40 mg/kg, 10 mL/kg), the mean observed Tmax was delayed (6.7 hours) in comparison with the other formulations. These results suggest an eventual saturation of absorption when the compound was formulated in Formulation 5.

Calculated half-life was comparable between the groups: after exclusion of the profiles when elimination phase of the curve was not accurately defined (AUCextra > 20% or Rsq <0.8), 6.3 hours was calculated for one subject receiving the Formulation 2 (IV); mean half-life was 4.7 hours (+-1.8) after oral administration of Formulation 3 and 4.3 hours ± 1 after administration of Formulation 4.

Taking into account the prolonged absorption phase with delayed Tmax after administration of Formulation 5, the number of points after the Cmax were not sufficient to accurately define the elimination curve.

After oral administration of buffer only to the control group, no compound was measured in blood samples, as expected.

### Part (v) - Investigation of liposome types (Cryo-TEM)

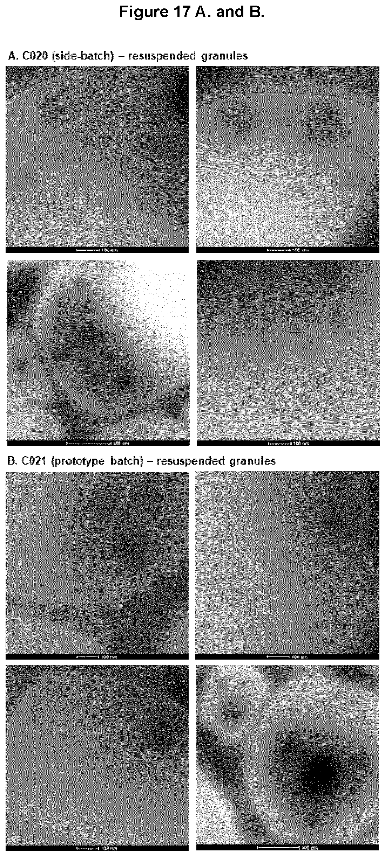

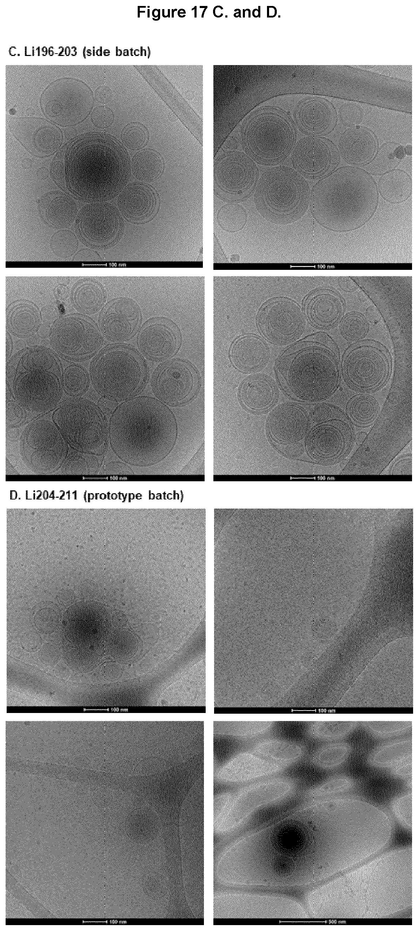

To acquire a better understanding as to why the side batch resulted in a higher bioavailability (22%) than the prototype batch (7.13%), the liposome type was further investigated. This was performed by means of cryogenic transmission electron microscopy (cryo-TEM, Talos 120 and Talos 200). Furthermore, to investigate how the coating and the drying method affect the liposomes, the granules used in the rat study, C020 and C021, were compared with the fresh liposomal dispersions Li196-203 and Li204-211, respectively. Representative micrographs are displayed in Figure 17.

All of the analysed samples contained a mixture of liposomes. The types of liposomes are unilamellar (ULV, liposome with a single bilayer), bilamellar (BLV, 2 concentric vesicles), oligolamellar (OLV, liposome with up to 3 concentric vesicles), multilamellar (MLV, liposome with more than 3 concentric vesicles), multivesicular (MVV, liposome with multiple nonconcentric lipid bilayers) and multilamellar-multivesicular liposomes (MLV-MVV, liposome with more than 3 concentric vesicles and multiple nonconcentric lipid bilayers) (Table 16).

According to the liposome distribution, C020 (side-batch) showed more multilamellar-multivesicular liposomes (35.5%) than the C021 (prototype, 19%). In both batches unilamellar liposomes accounted for half of the type of liposomes observed (48.2% and 58.4%, respectively). The rest of the liposome types observed showed comparable values between the two batches: bilamellar liposomes (2% and 5.3%, respectively), oligolamellar liposomes (0.8% and 0.6%, respectively) and multivesicular liposomes (13.39% and 16.5%, respectively). Overall, it appears that the obvious difference between the two batches lies in the higher amount of multilamellar-multivesicular liposomes in the side-batch (C020), than in the prototype batch (C021).

Moreover, when analysing their liposomal dispersions, a majority of 71% of multilamellar-multivesicular liposomes were observed in the prototype batch Li204-211, and 52.59% multilamellar-multivesicular liposomes in side-batch Li196-203. That makes more multilamellar-multivesicular liposomes in the liposomal dispersion than in granules. Consequently, more unilamellar liposomes were detected in granules than in the liposomal dispersion. This could indicate that, upon the coating process, the liposomes rehydrate into preferably unilamellar liposomes, and less multilamellar-multivesicular liposomes. Moreover, side batch layering preserves the multilamellar-multivesicular liposomes better than prototype layering.

Li204-211 (prototype) displayed more multilamellar-multivesicular liposomes (71%) than Li196-203 (side-batch) (52%). The opposite was observed for unilamellar liposomes (18.8% and 32%, respectively).

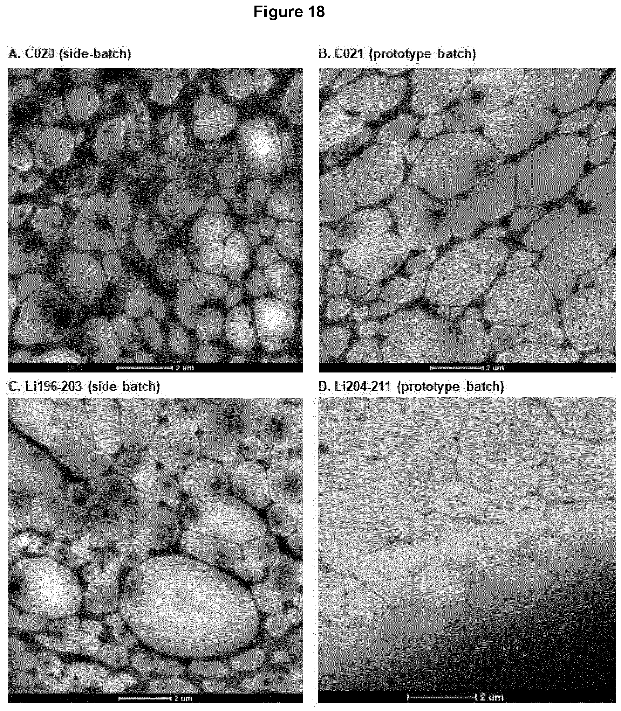

The median size of the liposomes was also analysed from cryo-TEM micrographs. As displayed in Table 12, unilamellar and multilamellar-multivesicular liposomes were screened. Overall, multilamellar-multivesicular liposomes were generally bigger than the unilamellar liposomes. However, the biggest liposomes were in most cases giant unilamellar vesicles (GUVs), sometimes containing additional vesicles (Figure 18). Giant vesicles containing multilamellar or multilamellar-multivesicular vesicles were infrequently observed. These results indicate several things: (1) multilamellar-multivesicular vesicles are generally bigger than unilamellar vesicles; (2) rehydrated liposomes from granules are bigger than the liposomes from the dispersion, which is an indication of their swelling upon restauration; (3) vesicles in the side-batch, both granules and dispersions, are bigger than the prototype vesicles, which reflects that the higher CBD:lipid ratio (0.7) shape into bigger liposomes than the lower ratio used in the prototype (0.09).

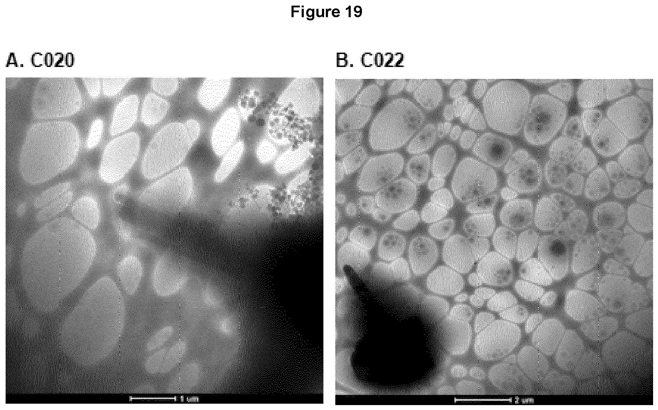

Additionally, crystals of approximately 4 - 9 µm were detected in C020 and Li196-203 (Figure 19). Their presence could indicate crystallized CBD, impurities or sugar. No crystals were detected in the prototype batches (C021 and Li204-211). That the crystals are only detected in the side-batch and not in the prototype could be a reflection of the higher CBD to lipid ratio 0.7 in comparison to 0.09, respectively.

In the light of the cryo-TEM results, it has been determined that the higher bioavailability of C020 in comparison to C021 is reflected by the increased amount and size of multilamellar-multivesicular liposomes.

This is contrary to conventional standards in liposome technology. Conventionally, one aims for smaller unilamellar liposomes with a narrow size distribution, which means that Group A as defined in Example 2 is most preferred, group B is less preferred and group C is least preferred. According to the invention, group A is least preferred, group B more preferred and group C most preferred. Therefore, unconventionally, one aims for liposomes with more than one bilayer, in particular multilamellar-multivesicular liposomes, larger liposomes and/or liposomes with a higher polydispersity index.

### Part (vi) - Investigation of liposome size (Zetasizer)

Samples were analysed with Zetasizer. The corresponding hydrodynamic diameter and PDI, together with their peaks are listed in Table 17.

### Example 6: Second rat study - liposomal dispersion

The results of the second rat study are also included in the general overview of rat studies and characterisation of prototype batches (Table 10). Details of the second rat study are as follows:
Upon the optimizations of the liposomal formulations after the first rat study (according to Example 2, subsection "Optimisation of CBD:lipid ratio and lipid type (after the first rat study)), a second rat study was conducted to test for the pharmacokinetic properties. The formulations were tested as liposomal dispersions.

As displayed in Table 18, seven study arms were prepared (F1-F7). Alike the first rat study, the 5 mg/kg intravenously administered formulation (Table 13) (arm number F1) was used for final calculations. For arms F2-F6, the 40 mg/kg of the following liposomal dispersions were orally fed (PO): "0.42" without and with cholesterol (F2 and F3, respectively), "0.7" without and with cholesterol (F4 and F5, respectively) and "1.3" without cholesterol (F6). The numbers in quotes correspond to the CBD:lipid ratio. Alike the first rat study, the commercial CBD-containing product Epidyolex was used as a reference as F7.

Three Sprague-Dawley rats were tested for each study arm. Concentrations and administered amount are displayed in Table 18. The samples were prepared four days before the application and the empirical CBD concentration was determined one day in advance (Table 5, Table 10, Table 19). The samples were sent in brown glass vials at 4° C and diluted to 4 mg/ml in 5% sucrose right before application.

### Part (i) - Analytic result of the formulations

The liposomal dispersions used in the second rat study were further analysed for their PDI, encapsulation efficiency and zeta potential. The values are resumed in Table 19.

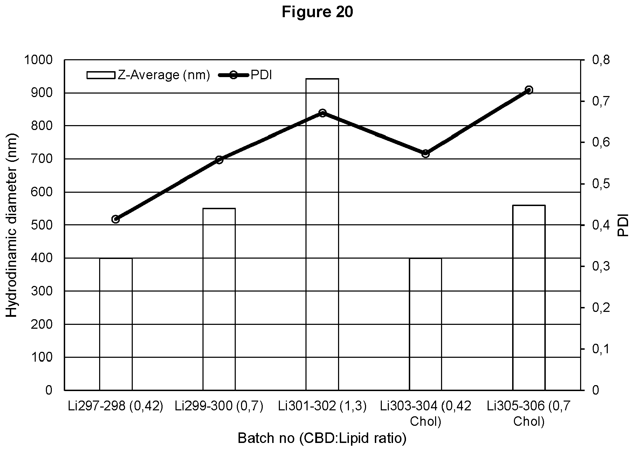

Further illustrated in a diagram (Figure 20), both the hydrodynamic diameter and the PDI increased with the amount of CBD in the absence and in the presence of cholesterol. That means the more CBD, the more heterogenous in size the liposomes are. Moreover, the opposite was observed for the zeta potential. For the samples without cholesterol, for a CBD: lipid ratio of 0.42 a value of -57.2 mV was acquired, for a ratio of 0.7 a value of -27 mV was acquired and for a ratio of 1.3, a value of -16.5 mV was acquired. For the cholesterol-containing samples, for a CBD:lipid ratio of 0.42 a value of -11.9 mV was acquired, and for 0.7 ratio a value of -17.3 mV was acquired. This indicates the more CBD reconstituted into the membranes, the higher the zeta potential value becomes.

Generally, the samples containing cholesterol had a higher zeta potential value, which indicates that lower CBD amounts were reconstituted into the membranes. This is also in line with the encapsulation efficiency results. That might be explained by the fact that cholesterol competes with CBD for reconstitution into the membranes, which leads to a lower CBD encapsulation efficiency.

### Part (ii) - Pharmacokinetic results

Pharmacokinetic parameters were obtained from mean blood concentrations by noncompartmental analysis using the software Phoenix WinNonLin^{®}.

After IV administration of Formulation 1 (F1 - 5 mg/kg) and oral administration of Formulations 2 to 6 (F2 to F6 - 40 mg/kg), the kinetics profiles were reproducible between the animals of the same group.

After administration of formulation "0.42" without cholesterol (Formulation 2) and with cholesterol (Formulation 3), the mean observed Cmax and exposure over time (AUC) were higher after administration of the formulation with cholesterol. The mean observed Tmax was slightly longer after administration of the formulation with cholesterol.

After administration of Formulation 2, the mean Cmax was 946 ng/mL and mean Tmax was 1.67 hours. After administration of Formulation 3, the mean Cmax was 1456 ng/mL and mean Tmax was 2.17 hours.

The estimated bioavailability (F%) was higher for the formulation with cholesterol (Formulation 3, mean F%: 65%) in comparison with the formulation without cholesterol (Formulation 2, F%: 51%)

After administration of Formulation "0.7" without cholesterol (Formulation 4) and with cholesterol (Formulation 5), the absorption phase was comparable between both formulations with similar mean observed Cmax and Tmax. The exposure over time (AUC) was slightly higher after administration of the formulation without cholesterol.

After administration of Formulation 4, the mean Cmax was 1598 ng/mL and mean Tmax was 2.17 hours. After administration of Formulation 5, the mean Cmax was 1538 ng/mL and mean Tmax was 2.67 hours.

The estimated bioavailability (F%) was slightly higher with the formulation without cholesterol (Formulation 4, meanF%: 83%) in comparison with the formulation with cholesterol (Formulation 5, mean F%: 69%).

After administration of Formulation "1.3" without cholesterol (Formulation 6), the kinetics profile was comparable to the profile obtained after administration of Formulation "0.7" with cholesterol (Formulation 5).

The mean Cmax was 1082 ng/mL and the mean Tmax was 2.67 hours.

The estimated bioavailability was similar to Formulation 5 (Formulation 6, mean F%: 67%)

Taken together, the mean observed Cmax and exposure over time (AUC) after administration of the oral formulations "0.42", "0.7" and "1.3" (Formulation 2 to 6), were around two fold higher in comparison with the commercial formulation (Formulation 7, mean Cmax: 460 ng/mL and mean AUC 6 398 h.ng/mL).

The mean Tmax was longer for the commercial formulation (Formulation 7, mean Tmax: 4 hours).

The bioavailability of the commercial formulation was lower (Formulation 7, mean F%: 32%) than formulations 2 to 6 (Formulations 2 to 6, mean F%: 50.83 to 82.98%).

Altogether, Examples 1 to 6 have demonstrated the following:
Nanoliposomes of CBD with DMPC with and without cholesterol and CBD loadings of 10 to 130% could be made on a lab scale with a dual centrifuge. Three batches (229717LN001-003) were attempted for scale up to 780, 830 and 960 g in a Netzsch Nanomill.

The liposomes were stabilized with sucrose and layered onto isomalt starter particles in a MiniGlatt fluid bed process (C001-C017, C020, C021) on a 10 g scale. The first scale up to layer a larger amount in an Unilab (C018) failed due to severe process issues and the second attempt (C019) led to strong agglomeration of the product.

The granules from batches C020 (side batch) and C021 (prototype batch) were tested in a first rat study vs CBD administered i.v. and the marketed reference product (Epidyolex). Granules from the side batch showed higher bioavailability (22%, arm F4) than the ones from the prototype batch with the same 40 mg CBD / kg rat concentration (12%, arm F3). Tmax was higher in the granules (0.5 - 1.8 h) than the commercial product Epidyolex (6.6 h).

Liposomes from the granules and liposomal dispersion were further characterized via cryo-TEM. The side batch displayed a higher number of multilamellar-multivesicular liposomes (35.4%) than the prototype batch (19%). Their corresponding liposomes in the liposomal dispersions appeared generally smaller and more multilamellar-multivesicular before layering (52% and 71%, respectively).

Liposomal formulations were further optimized in different CBD concentrations 25%, 42%, 50% (with cholesterol) and 42%, 70%, 80%, 100% (without cholesterol), respectively, and analysed via cryo-TEM.

A selection of five liposomal formulations in different CBD concentrations 42%, 70% (with cholesterol), and 42%, 70%, 130% (without cholesterol) were further tested in a second rat study, respectively. CBD *i.v* and the marketed reference product (Epidyolex) were also administered, respectively. The results showed overall notably improved bioavailability of the liposomal formulation. The results were two folds higher than the marketed product (31.8%). The batch 70% CBD-without cholesterol (arm F4) showed the highest bioavailability (82.9%) of all the liposomal dispersions tested. Cholesterol-containing samples (arms F3 and F5) had no advantage over the samples without cholesterol (arms F2, F4, F6).

### List of Tables

| | |
|---|---|
| **Table** 1: | API (Tab. 1A) and excipients (Tab. 1B) used. |
| **Table 2:** | Parameters used for the optimization of the lipid formulation and their corresponding analytic results. |
| **Table 3:** | Parameters used for the optimization of the lipid formulation and their corresponding analytic results. |
| **Table 4:** | Parameters used for the optimization of the lipid formulation and their corresponding analytic results. |
| **Table 5:** | Parameters used for the optimization of the lipid formulation and their corresponding analytic results (after the first rat study). |
| **Table 6:** | Parameters used for the optimization of the lipid formulation and their corresponding analytic results (after the first rat study). |
| **Table 7:** | Parameters used for the optimization of the lipid formulation and their corresponding analytic results (after the first rat study). |
| **Table 8:** | Parameters of C001-C021. |
| **Table 9:** | Parameters used for the lipid formulation prepared with the bead mill and their corresponding analytic results. |
| **Table 10:** | Summary of the rat studies and characterization of prototype batches. |
| **Table 11:** | Z-sizer results from 229717C017 and 229717C018 tested batches ddh2o-dispersed granules. |
| **Table 12:** | Z-sizer results from batches 229717C017 (C017), 229717C020 (C020), 229717C021 (C021) dispersed in 5% sucrose. |
| **Table 13:** | Tabular overview of study arms and preparations. |
| **Table 14:** | Parameters determined for granules dispersions used in the first rat study (229717C020 and 229717C021), such as encapsulation efficiency (EE%), hydrodynamic diameter, PDI and zeta potential. The expected CBD:lipid ratio was calculated as reflecting the real CBD:lipid ratio, considering that all |
| | phospholipids are reconstituted into liposomes. It was calculated as following: (CBD:Lipid ratio * (EE%/100)) |
| **Table 15:** | Pharmacokinetic parameters. Cmax stands for maximum concentration of CBD after dosing, Tmax stands for time to peak drug concentration, AUC stands for area under the concentration-time curve. |
| **Table 16:** | Distribution of liposome-type (%) retrieved from cryo-TEM micrographs of batches C020-023. |
| **Table 17:** | Particle size distribution of the 2 months old granule batches C020, C021, and fresh liposomal dispersions Li196-203, Li204-211 analysed with Zetasizer |
| **Table 18:** | Arms with the formulations used in the second rat study. |
| **Table 19:** | Parameters determined for the liposomal dispersions used in the second rat study (229717Li297-306), such as encapsulation efficiency (EE%), hydrodynamic diameter, PDI and zeta potential. The expected CBD:lipid ratio was calculated as reflecting the real CBD:lipid ratio, considering that all phospholipids are reconstituted into liposomes. It was calculated as follows: (CBD:lipid ratio * (EE%/100)) |
| **Table 20:** | Pharmacokinetic results obtained in the second rat study (Formulation 1). |
| **Table 21:** | Pharmacokinetic results obtained in the second rat study (Formulations 2 and 3). |
| **Table 22:** | Pharmacokinetic results obtained in the second rat study (Formulations 4 and 5). |
| **Table 23:** | Pharmacokinetic results obtained in the second rat study (Formulations 6 and 7). |
| **Table 24:** | Parameters of the batches C018 and C019 of the spraying trials using Unilab. |

### List of Figures

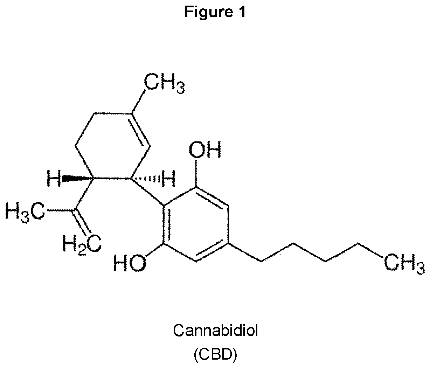

- **Figure 1:**: Chemical structure of cannabidiol (CBD).
- **Figure 2:**: Cryo-TEM micrographs of liposomal dispersions from batches Li249-250, Li251-252, Li253-254and Li255-256, where DMPC alone was used as a lipid source. Two representative micrographs are displayed for each batch. The label assigned on top of them i.e. "Li249-250 / 0.42" corresponds to "Batch number / CBD:lipid ratio". Samples were prepared as follows: liposome dispersions with a lipid concentration of 91 mg/ml were diluted to 3 mg/ml in Milli-Q right before freezing on the grid. 3 µl of each sample were applied on a carbon-sputtered copper grid covered with a perforated polymer film. The liquid excess was removed from the grid by blotting it against a filter paper. The remaining liposome film formed on the grid was immediately frozen in liquid ethane and kept below -160° C until transferred to a microscope). All micrographs are depicted on a 100 nm scale.
- **Figure 3:**: Graph depicting the quantification of DMPC-containing liposomes from batches Li249-250, Li251-252, Li253-254 and Li255-256 from cryo-TEM micrographs. The label assigned below them i.e. "Li249-250 / 0.42" correspond to "Batch number / CBD:lipid ratio". Batches Li196-203 (side-batch) and Li204-211 (prototype batch) were added for comparison from previous cryo-TEM micrographs (see Figure 18).
- **Figure 4:**: Cryo-TEM micrographs of liposomal dispersions from batches Li257-258, Li259-260, Li261-262 and Li263-264, where 80% DMPC and 20% Cholesterol were used as lipid source. Two representative micrographs are displayed for each batch. The label assigned on top of them i.e. "Li257-258 / 0.25" correspond to "Batch number / CBD:lipid ratio". Samples were prepared as described before (see legend to Figure 2). All micrographs are depicted on a 100 nm scale.
- **Figure 5:**: A. Graph depicting the quantification of 80% DMPC and 20% cholesterol-containing liposomes from batches Li257-258, Li259-260, Li261-262 and Li263-264 from cryo-TEM micrographs. The label assigned below them i.e. "Li257-258 / 0.25" correspond to "Batch number / CBD:lipid ratio". Batches Li196-203 (side-batch) and Li204-211 (prototype batch) were added for comparison from previous cryo-TEM micrographs (see Figure 18). **B.** 229717LN001. Scale-up with bead mill, silibeads Zy-S, size: 0.8 mm.
- **Figure 6:**: 229717LN002. Scale-up with bead mill, silibeads Zy-S, size: 0.5 mm.
- **Figure 7:**: 229717LN003. Scale-up with bead mill, silibeads Zy-S, size: 0.5 mm.
- **Figure 8:**: Hydrodynamic diameter and PDI after various amounts of layered CBD per g carrier (Batch: 229717C005). The data point at x=0 corresponds to liposome dispersion.

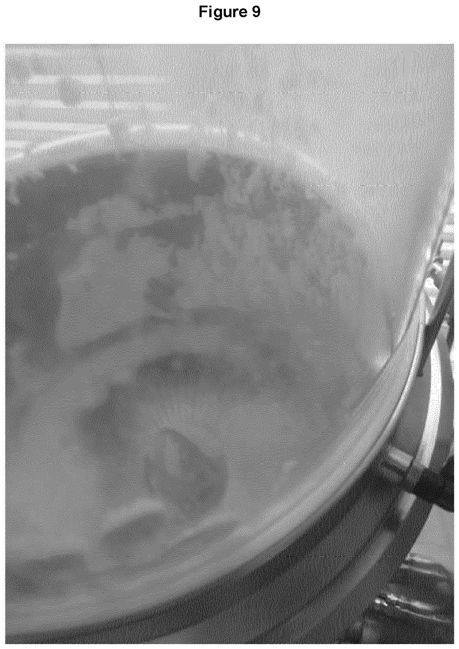

- **Figure 9:**: Agglomeration of product in fluid bed system.
- **Figure 10:**: Sediment formation of water dispersed granulate from batch 229717C018.
- **Figure 11:**: Granules from batch 229717C017 containing 1 mg/ml, 2 mg/ml and 4 mg/ml CBD, respectively, were Mill-Q-dispersed by 10 min stirring at room temperature. The dispersion was allowed to settle for 10 min, upon which no sediment formation was observed.
- **Figure 12:**: Hydrodynamic diameter (Z-Average) and PDI results of CBD granules dissolved in ddH2O or 5% Succrose. 1 mg/ml, 2mg/ml and 5 mg/ml CBD-containing granules were dissolved in ddH2O and 5% sucrose, respectively, and measured with the Z-sizer. Left: Z-Average results; Right: Pdl results.

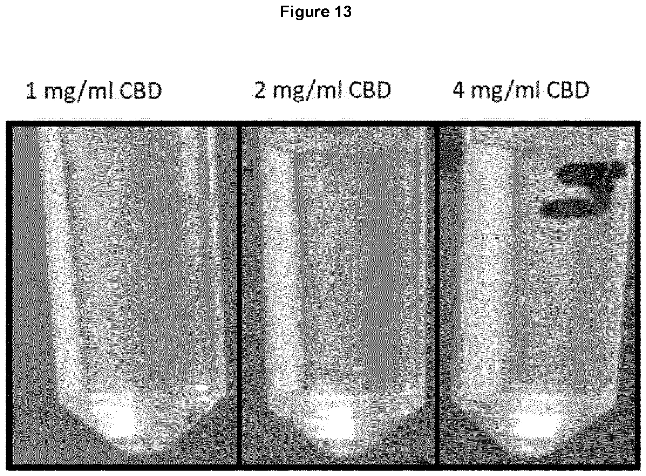

- **Figure 13:**: Commercial CBD diluted in sesame oil. 0.01 ml, 0.02 ml and 0.4 ml of Epydiolex were mixed with up to 1 ml sesame oil, respectively, to reach 1 mg/ml, 2 mg/ml and 4 mg/ml, respectively. Left: 1 mg/ml CBD in sesame oil; middle: 2 mg/ml CBD in sesame oil; Right: 4 mg/ml CBD in sesame oil.
- **Figure 14:**: Commercial CBD diluted to 4 mg/ml in two solvents: **A.** Sesame oil: 0.6 ml 100 mg/ml CBD Epidyolex was added to 14.4 ml and stirred for 10 min at room temperature. **B.** 9:1 sesame oil: ethanol absolute: 18 ml sesame oil was mixed with 2 ml ethanol absolute. 14.4 ml of this mixture was further mixed with 0.6 ml 100 mg/ml CBD Epidyolex. Top: side view of the suspensions in the glass. Bottom: top view of the suspensions in the glass.
- **Figure 15:**: Injection formulations. Left: first approach for which 50 mg/ml CBD was dissolved in 95% Ethanol and further diluted to 1 mg/ml CBD in 96% saline and 2% Tween 80. Right: second approach for which 50 mg/ml CBD was dissolved in 1:1 Ethanol : Tween 80 and further diluted to 1 mg/ml CBD in 98% saline. **A.** Formulation upon dilution; **B.** First approach - formulation after 10 min stirring compared to second approach without stirring; C.First approach - formulation after 10 min heat stirring compared to second approach without heat stirring. "Fat drops" are indicated with arrows. A magnet was added in the second approach formulation for comparison.
- **Figure 16:**: Injection formulation. 50 mg/ml CBD was dissolved in 0.2 ml ethanol absolute and further diluted to 5 mg/ml CBD in sesame oil, reaching a final ratio of sesame oil:ethanol of 9:1
- **Figure 17:**: Representative micrographs of liposomes from the batches C020, C021, Li196-203, Li204-211. Granules from batches C020 (A) and C021 (B) were dissolved in Milli-Q in Eppendorf tubes by 10 min end-to-end mixing to reach a final concentration of 3 mg/ml lipids, whilst liposome dispersions from batches Li196-203(C) and Li204-211 (D) with a lipid concentration of 91 mg/ml were diluted in Milli-Q to 3 mg/ml right before freezing on the grid. 3 µl of each sample were applied on a carbon-sputtered copper grid covered with a perforated polymer film. The liquid excess was removed from the grid by blotting it against a filter paper. The remaining liposome film formed on the grid was immediately frozen in liquid ethane and kept below -160° C until transferred to a microscope. Micrographs were retrieved with a Talos 120 transmission electron microscope for (A) and Talos 200 transmission electron microscope for (B, C, D).
- **Figure 18:**: Cryo-TEM micrographs of the batches C020, C021, Li196-203, Li204-211 (A-D). The samples were prepared as described above. Red arrows indicate giant unilamellar vesicles, whilst the blue arrows indicate giant multilamellar vesicles.
- **Figure 19:**: Crystals present in micrographs of batch C020 (side-batch granules) (A) and batch Li196-203 (side batch liposomal dispersion) (B). Micrographs were retrieved as described above.
- **Figure 20:**: Diagram illustrating the hydrodynamic diameter (nm) (Hydrodynamic diameter) and PDI of the liposomal dispersions tested in the second rat study. The bar charts indicate the hydrodynamic diameter and the line indicates the PDI.

**Table 1**

| **A) API** | | | | | | |
|---|---|---|---|---|---|---|
| **Product** | **Article No.** | **Batch** | **Amount** | **Distributor** | | **Manufacturer** |
| Canapure (cannabidiol synthetically produced) | 800023 | 19Y21747 | 1 kg | Arevipharma | | Symrise |
| | 689383 | 10300018 | | | | |

| **B) Excipients** | | | | | | |
|---|---|---|---|---|---|---|
| **Excipient** | | **Grade** | **Manufacturer** | | **Function** | |
| Soybean Phosphatidylcholine from Soybean | | S-100 | Lipoid GmbH | | Phospholipid component (zwitterionic) | |
| Hydrogenated Phosphatidylcholine from Soybean (Phospholipon 90H) | | P 90H | Lipoid GmbH | | Phospholipid component (zwitterionic) | |
| 1,2-Dimyristol-sn-glycerol-3-phosphocholine (DMPC), Lipoid PC 14:0/14:0 | | DMPC | Lipoid GmbH | | Phospholipid component (zwitterionic) | |
| Cholesterol | | Chol | Lipoid GmbH | | Sterol component | |
| Isomalt 720 | | GalenIQ^{™} 720 | BENEO-Palatinit GmbH | | Solid carrier material | |

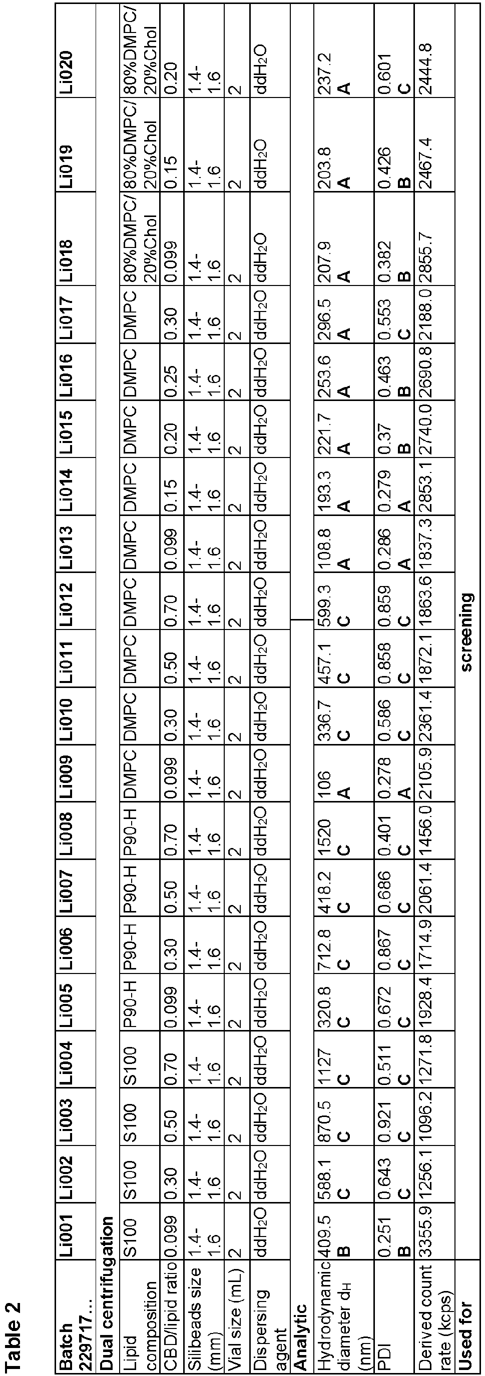

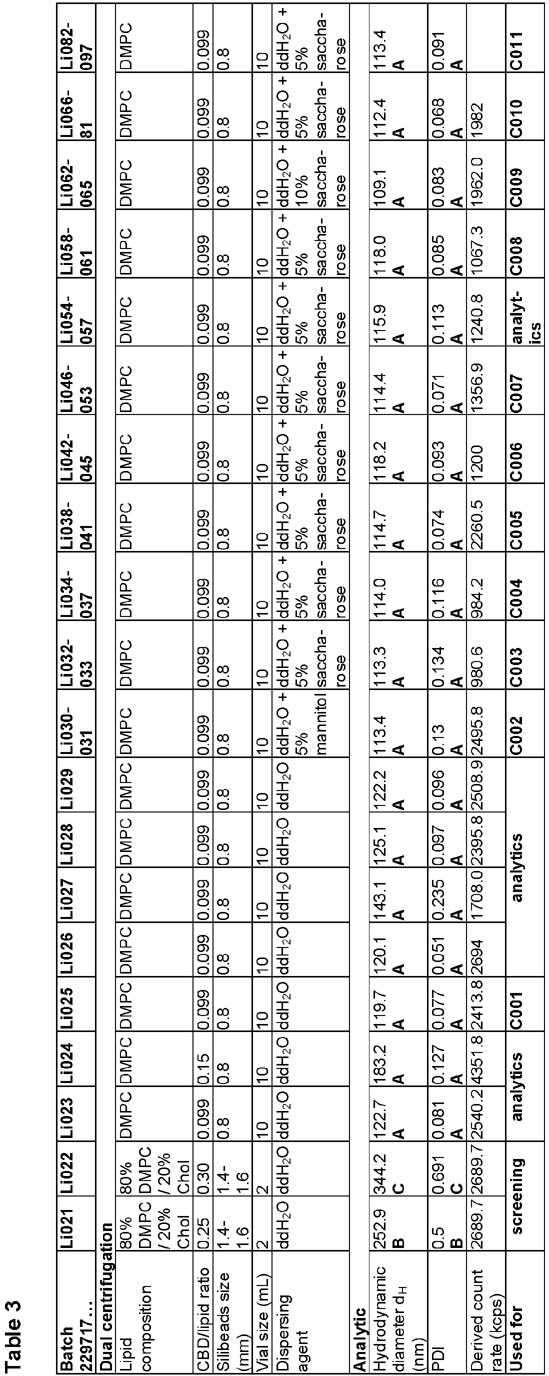

**Table 4**

| **Batch 229717...** | **Li097-113** | **Li114** | **Li115-118** | **Li119-122** | **Li123-126** | **Li127-142** | **Li143-158** | **Li163-178** | **Li179-195** | **Li196-203** | **Li204-211** |
|---|---|---|---|---|---|---|---|---|---|---|---|
| **Dual centrifugation** | | | | | | | | | | | |
| Lipid composition | DMPC | DMPC | DMPC | DMPC | DMPC | DMPC | DMPC | DMPC | DMPC | DMPC | DMPC |
| CBD/lipid ratio | 0.099 | 0.701 | 0.701 | 0.701 | 0.701 | 0.701 | 0.099 | 0.701 | 0.099 | 0.701 | 0.099 |
| Silibeads size (mm) | 0.8 | 1.4-1.6 | 0.8 | 0.8 | 0.8 | 0.8 | 0.8 | 0.8 | 0.8 | 0.8 | 0.8 |
| Vial size (mL) | 10 | 2 | 10 | 10 | 10 | 10 | 10 | 10 | 10 | 10 | 10 |
| Dispersing agent | ddH₂O + 5% saccharose | ddH₂O + 5% saccharose | ddH₂O + 5% saccharose | ddH₂O + 5% saccharose | ddH₂O | ddH₂O + 5% saccharose | ddH₂O + 5% saccharose | ddH₂O + 5% saccharose | ddH₂O + 5% saccharose | ddH₂O + 5% saccharose | ddH₂O + 5% saccharose |

| **Analytic** | | | | | | | | | | | |
|---|---|---|---|---|---|---|---|---|---|---|---|
| Hydrodynamic diameter d_{H} (nm) | 117.6 | 440.3 | 484.0 | 518.4 | 562.8 | 523.3 | 116.4 | 512.0 | 116.0 | 512.3 | 149.8 |
| | A | C | C | B | B | B | A | C | A | B | A |
| PDI | 0.097 | 0.739 | 0.533 | 0.384 | 0.384 | 0.486 | 0.081 | 0.749 | 0.095 | 0.413 | 0.154 |
| | A | C | C | B | B | B | A | C | A | B | A |
| Derived count rate (kcps) | 3421.7 | 1822.2 | 1806.5 | 1822.2 | 1988.7 | 1862.3 | 1583.1 | | 515.3 | 1714.1 | 2340.3 |
| Used for | C012 | zetasizer | C013 | C014 | C015 | C016 | C017 | C020 | C021 | Cryo-TEM | Cryo-TEM |

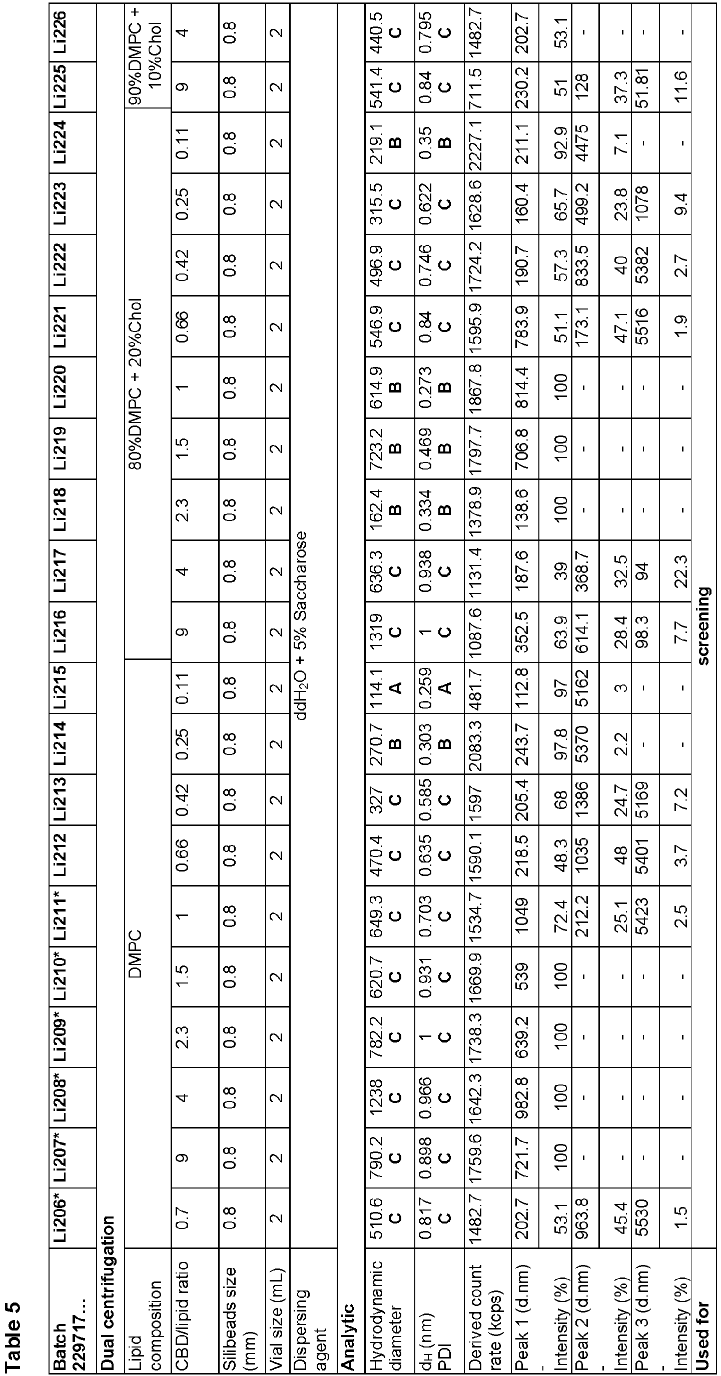

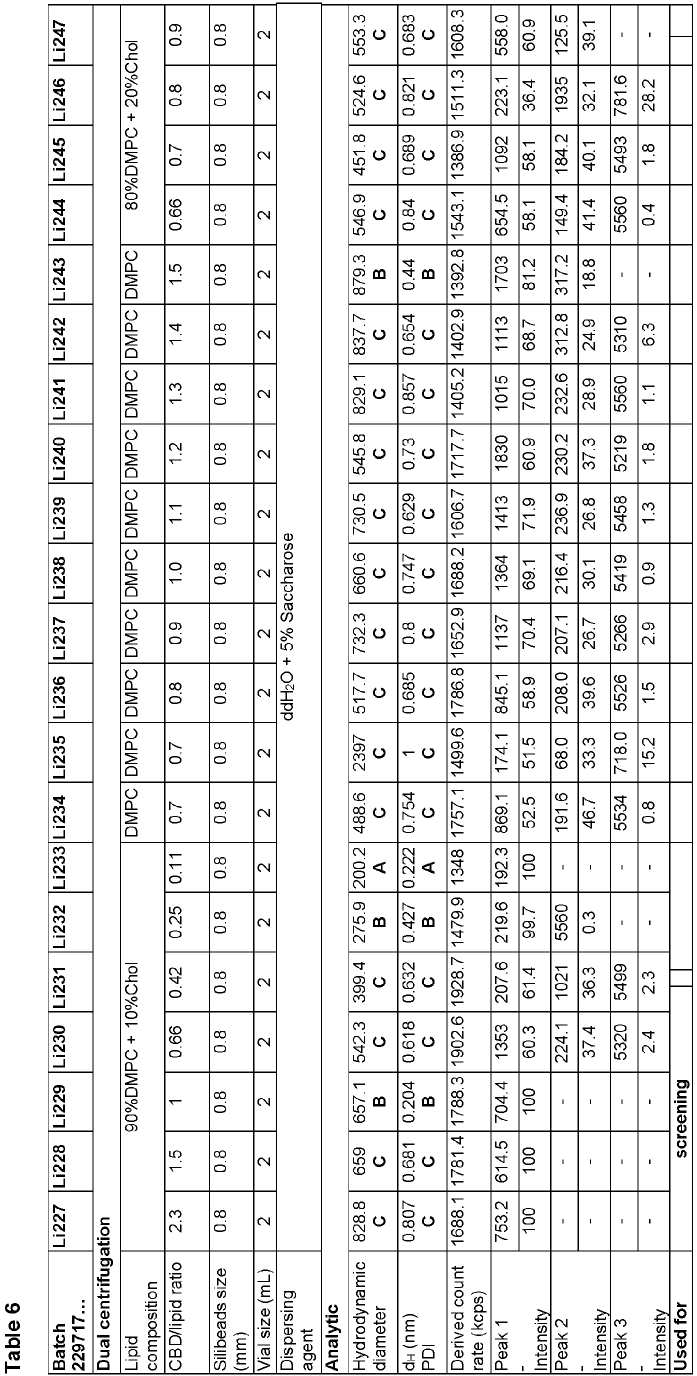

**Table 7**

| **Batch 229717...** | **Li248** | **Li249-250** | **Li251-252** | **Li253-254** | **Li255-256** | **Li257-258** | **Li259-260** | **Li261-262** | **Li263-264** | **Li297-298** | **Li299-300** | **Li301-302** | **Li303-304** | **Li305-306** |
|---|---|---|---|---|---|---|---|---|---|---|---|---|---|---|
| **Dual centrifugation** | | | | | | | | | | | | | | |
| Lipid composition | 80%DMPC/ 20%Chol | DMPC | DMPC | DMPC | DMPC | 80%DMPCI 20%Chol | | | | DMPC | DMPC | DMPC | 80%DMPC/ 20%Chol | |
| CBD/lipid ratio | 1.0 | 0.42 | 0.7 | 0.8 | 1.3 | 0.25 | 0.42 | 0.5 | 1.0 | 0.42 | 0.7 | 1.3 | 0.42 | 0.7 |
| Silibeads size (mm) | 0.8 | 0.8 | 0.8 | 0.8 | 0.8 | 0.8 | 0.8 | 0.8 | 0.8 | 0.8 | 0.8 | 0.8 | 0.8 | 0.8 |
| Vial size (mL) | 2 | 10 | 10 | 10 | 10 | 10 | 10 | 10 | 10 | 10 | 10 | 10 | 10 | 10 |
| Dispersing agent | ddH₂O + 5% Saccharose | | | | | | | | | | | | | |

| **Analytic** | | | | | | | | | | | | | | |
|---|---|---|---|---|---|---|---|---|---|---|---|---|---|---|
| EE (%) | - | 93 | 92 | 88 | 78 | 81 | 77 | 65 | 61 | 94 | 91 | 87 | 59 | 58 |
| Amount of CBD (mg/g granules) | | 51.6 | 73.1 | 70.7 | 103.1 | 84.0 | 33.9 | 45.7 | 71.1 | 48.5 | 66.1 | 90.7 | 40.8 | 57.7 |
| Hydrodynamic diameter d_{H} (nm) | 524.4 C | 371.3 B | 491.8 C | 547.3 C | 779.9 C | 673.8 B | 426.5 C | 727 C | 644.9 C | 398.8 B | 550.3 C | 941.5 C | 399.6 C | 559.2 C |
| PDI | 0.819 C | 0.366 B | 0.534 C | 0.614 C | 0.783 C | 0.315 B | 0.558 C | 0.756 C | 0.802 C | 0.414 B | 0.558 C | 0.671 C | 0.573 C | 0.727 C |
| Derived count rate (kcps) | 1247.4 | 2161.9 | 1673.3 | 1643 | 1454.3 | 1804.1 | 2276.6 | 1634.3 | 1577.4 | 398.8 | 550.3 | 941.5 | 399.6 | 559.2 |
| Peak 1 Intensity | 1487 | 309.7 | 357.8 | 330.0 | 1265 | 761.8 | 279.3 | 1083 | 381.4 | 342.2 | 330.9 | 1551 | 242.1 | 813.7 |
| | 73.2 | 96.7 | 79.2 | 70.6 | 70.0 | 91.1 | 76.9 | 58.6 | 86.6 | 92.7 | 64.6 | 43.7 | 82.0 | 50.7 |
| Peak 2 Intensity | 238.2 | 5251 | 986.0 | 1086 | 249.6 | 211.8 | 1618 | 249.0 | 1276 | 4860 | 1404 | 623.9 | 749.3 | 231.1 |
| | 25.5 | 3.3 | 17.7 | 26.6 | 28.7 | 4.9 | 14.1 | 41.4 | 12.5 | 7.3 | 30.8 | 38.7 | 16.9 | 45.9 |
| Peak 3 Intensity | 5252 | - | 5427 | 5469 | 5453 | 4849 | 5179 | - | 5560 | 0 | 5238 | 271.5 | 5541 | 5420 |
| | 1.3 | - | 3.1 | 2.8 | 1.4 | 3.9 | 9.0 | - | 0.8 | 0 | 4.6 | 15.6 | 10 | 3.3 |
| **Used for** | **zetasizer** | **Cryo-TEM, EE(%)** | | | | | | | | **Rat study, EE%, Zetasizer** | | | | |

**Table 8**

| **Batch 229717...** | **C001** | **C002** | **C003** | **C004** | **C005** | **C006** | **C007** | **C008** | **C009** | **C010** | **C011** | **C012** | **C013** | **C014** | **C015** | **C016** | **C017** | **C020** | **C021** |
|---|---|---|---|---|---|---|---|---|---|---|---|---|---|---|---|---|---|---|---|
| Used liposome dispersion | Li025 | Li 030-LiO31 | Li032-Li033 | Li034-Li037 | Li038-041 | Li042-045 | Li046-053 | Li058-061 | Li062-065 | Li066-081 | Li082-097 | Li 098-113 | Li115-118 | Li119-122 | Li 123-126 | Li127-142 | Li143-158 | Li163-178 | Li 179-195 |
| Dispersing agent | ddH₂O | ddH₂O + 5% Mannitol | ddH₂O + 5% Saccharose | | | | | | | | | | | | | | | | |
| CBD concentration of sprayed dispersion [mg/ml] | 900 | 9.01 | 900 | 15.00 | 9.03 | 900 | 900 | 900 | 900 | 8.87 | 900 | 9.00 | 63.77 | 63.77 | 63.78 | 63.76 | 9.00 | 63.76 | 9.0 |
| Lipid concentration of sprayed dispersion [mg/ml] | 90.97 | 91.08 | 91.03 | 151.67 | 91.32 | 91.01 | 91.00 | 91.00 | 91.00 | 89.66 | 91.00 | 91.00 | 91.01 | 91.01 | 91.03 | 91.00 | 91.00 | 91.00 | 91 |
| Final amount CBD per g carrier [mg/g] | 8.19 | 11.27 | 13.50 | 15.75 | 15.90 | 10.00 | 10.01 | 10.00 | 3.64 | 11.82 | 12.00 | 12.00 | 69.41 | 85.45 | 85.19 | 85.19 | 12.00 | 85.06 | 12 |
| Final amount lipid per g carrier [mg/g] | 82.78 | 113.94 | 136.55 | 159.25 | 160.72 | 101.11 | 101.09 | 101.09 | 36.81 | 119.52 | 121.34 | 121.37 | 99.06 | 121.96 | 121.59 | 121.58 | 121.33 | 121.4 | 121.33 |
| Batch size [g Isomalt 720] | 10 | 10 | 10 | 20 | 20 | 20 | 40 | 20 | 20 | 100 | 100 | 97.11 | 13.00 | 15.00 | 1400 | 60.00 | 100.00 | 58.00 | 100.00 |
| EE (%) | - | - | - | - | - | - | - | - | - | - | - | - | - | - | - | - | - | 61 | 97 |
| Amount CBD mg/g granulat | - | - | - | - | - | - | - | - | - | - | - | - | - | - | - | - | - | 61.2 | 9.18 |
| Hydrodynamic diameter d_{H} (nm) | 235.2 C | 253.0 C | 170.0 C | 567.2 B | 270.5 C | 188.8 B | 193.3 C | 237.5 C | 129 A | 242.3 B | 188.2 B | 176.9 B | 511.3 B | 526.4 A | 621.5 B | 528.5 B | 192.4 B | 2024 B | 1604 B |
| PDI | 0.543 C | 0.690 C | 0.504 C | 0.433 B | 0.665 C | 0.428 B | 0.56 C | 0.56 C | 0.237 A | 0.482 B | 0.438 B | 0.43 B | 0.457 B | 0.175 A | 0.37 B | 0.343 B | 0.487 B | 0.377 B | 0.291 B |
| Attenuator | 11 | 9 | 9 | 10 | 9 | 9 | 9 | 9 | 9 | 9 | 9 | 9 | 9 | 9 | 9 | 9 | 9 | 9 | 9 |

**Table 9**

| **Batch 229717...** | **LN001** | **LN002** | **LN003** |
|---|---|---|---|
| | | | |
| Lipid composition | DMPC | DMPC | DMPC |
| CBD/lipid ratio | 0.099 | 0.099 | 0.099 |
| Silibeads size (mm) | 0.8 | 0.5 | 0.5 |
| Batch size (mL) | 780 | 830 | 960 |
| Dispersing agent | ddH₂O | ddH₂O | ddH₂O |
| | + 5% saccharose | + 5% saccharose | + 5% saccharose |
| Start CBD concentration (mg/g) | 15.00 | 12.2 | 12.2 |
| Start lipid concentration (mg/g) | 151.7 | 123.2 | 123.2 |
| End CBD concentration (mg/g) | 12.2 | 12.2 | 12.2 |
| End lipid concentration (mg/g) | 123.2 | 123.2 | 123.2 |
| Total milling time (min) | 320 | 330 | 330 |
| Max agitator speed (rpm) | 2350 | 2480 | 2150 |
| Speed pump (rpm) | 185 | 195 | 165 |
| | Analytic results | | |
| Hydrodynamic diameter d_{H} (nm) | 132.0 **B** | 125.4 **A** | 165.4 **B** |
| PDI | 0.364 | 0.277 | 0.341 |
| | **B** | **A** | **B** |
| Derived count rate (kcps) | | | |
| Used for | **C018** | **C019** | **screening** |

**Table 10**

| | | | **Development phase** | | | | | | | | **Analytic** | | | | |
|---|---|---|---|---|---|---|---|---|---|---|---|---|---|---|---|
| Rat study | Arm no. | Lipid | Formulation | Batch no. | CBD: Lipid ratio | Concentration CBD/ rat (mg/kg) | AUC (h*kg*ng/ mL/mg) | Cmax (ng/ml) | Tmax (h) | F(%) | Encapsulation efficiency (%) | Assay purity (mg CBD/g dispersion) | Hydrodynamic diameter (d.nm.) | PDI | Z-potential |
| I | F1 =Intravenous CBD | - | 5 mg/mL CBD in 1:1:20 Ethanol:Tween 80:saline | - | - | - | 1026,8 | 6719,4 | 0.08 | - | - | - | - | - | - |
| | F2 = Prototype batch | DMPC | 4 mg/mL CBD (from granules) in ddH2O | C021 | 0.1 | 20 | 73 | 309.71 | 0.50 | 7.13 (SD: 3.33) | 97 | 9.18 | 116 **A** | 0.095 **A** | - |
| | F3 = Prototype batch | DMPC | | C021 | 0.1 | 40 | 127 | 651.28 | 0.50 | 12.32 (SD: 2.65) | 97 | 9.18 | 116 **A** | 0.095 **A** | - |
| | F4 =Side batch | DMPC | | C020 | 0.7 | 40 | 233 | 1158.2 | 1.83 | 22.73 (SD: 8.9) | 61 | 61.2 | 512 **C** | 0.749 **C** | - |
| | F5 = commercial CBD (Epydiolex) | - | 4 mg/mL CBD in Sesame oil | - | - | 40 | 209 | 401.9 | 6.67 | 20.36 (SD: 20.26) | - | - | - | - | - |
| | F6 = Negative control | - | ddH2O | - | - | - | - | - | | - | - | - | - | - | - |
| | | | | | | | | | | | | | | | |
| II | F1 =Intravenous CBD | - | 5 mg/mL CBD In 1:1:20 Ethanol:Tween 80:Saline | - | - | - | 502,19 | 2908 | 0.08 | - | - | - | - | - | - |
| | F2="0.42", without cholesterol | DMPC | 4 mg/ml CBD (from liposomal suspension) in 5% sucrose solution | 229717 Li297-298 | 0.42 | 40 | 255,28 | 946 | 1.67 | 50.83 (SD: 14) | 94 | 48.5 | 398,8 **B** | 0,414 **B** | -57,2 |
| | F3="0.42", with cholesterol | 80% DMPC + 20% ChoI | | 229717 Li303-304 | 0.42 | 40 | 326,90 | 1456 | 2.17 | 6509 (SD: 29.69) | 59 | 40.8 | 399,6 **C** | 0,573 **C** | -11,9 |
| | F4="0.7", without cholesterol | DMPC | | 229717 Li299-300 | 0.7 | 40 | 416,70 | 1598 | 2.17 | 82.98 (SD: 12.16) | 91 | 66.1 | 550,3 **C** | 0,558 **C** | -21,5 |
| | F5= "0.7", with cholesterol | 80% DMPC + 20% Chol | | 229717 Li305-306 | 0.7 | 40 | 345,43 | 1538 | 2.67 | 68.79 (SD: 25.55) | 58 | 57.7 | 559,2 **C** | 0,727 **C** | -17,3 |
| | F6= "1.3", without cholesterol | DMPC | | 229717 Li301 -302 | 2.3 | 40 | 334,88 | 1082 | 2.67 | 66.68 (SD 11.01) | 87 | 90.7 | 941,5 **C** | 0,671 **C** | -16,5 |
| | F7= Commercial CBD (Epidyolex) | - | 4 mg/ml CBD in sesame oil | - | - | 40 | 159.95 | 460.45 | 4 | 31.85 (SD 1.43) | - | - | - | - | - |

**Table 11**

| **Batch** | **Dispersant** | **CBD concentration** | **Sediment after 10 min rest** | **Hydrodynamic diameter d.nm.** | **Pdl** |
|---|---|---|---|---|---|
| C017 | ddH₂O | 1 mg/ml | no | 206 | 0.467 |
| | | 2 mg/ml | no | 204.3 | 0.438 |
| | | **4 mg/ml** | no | 180.2 | 0.326 |
| C018 (after stirring) | | 1 mg/ml | Yes | 1266 | 1.000 |
| | | 2 mg/ml | Yes | 1822 | 1.000 |
| | | 4 mq/ml | yes | 962.2 | 0.965 |
| C018 (1 h settling) | | 1 mg/ml | Yes | 494.9 | 0.859 |
| | | 2 mg/ml | Yes | 264.7 | 0.597 |
| | | 4 mg/ml | yes | 674.2 | 0.931 |
| C018 (1 µm filtrated) | | 1 mg/ml | No | 266.2 | 0.546 |
| | | 2 mg/ml | Slightly (filter broke) | 291.9 | 0.62 |
| | | 4 mg/ml | Slightly (filter broke) | 1113 | 1.000 |

**Table 12**

| **Batch** | **Solvent** | **CBD Concentration (mg/ml)** | **Sediment formation after 10 min rest** | **Hydrodynamic diameter d.nm.** | **Pdl** |
|---|---|---|---|---|---|
| C017 | ddH₂O | 4 | No | 194.4 **B** | 0.372 **B** |
| | 5% Sucrose | 1 | | 221.4 **B** | 0.497 **B** |
| | | 2 | | 206.5 **B** | 0.458 **B** |
| | | 4 | | 194.3 **B** | 0.366 **B** |
| C020 (theoretical load 85.1 mg/g CBD/carrier) | ddH₂O | 4 | Yes | 831.5 **C** | 0.952 **C** |
| | 5% Sucrose | 4 | | 649.3 **C** | 0.950 **C** |
| C021 (theoretical load 12.0 mg/g CBD/carrier) | ddH₂O | 4 | No | 152.8 **A** | 0.208 **A** |
| | 5% Sucrose | 4 | | 148.2 **A** | 0.255 **A** |
| C020 (adjusted to determined load 61.2 mg/g CBD/carrier) | ddH₂O | 4 | Yes | 990.9 **C** | 0.934 **C** |
| C021 (adjusted to determined load 9.18 mg/g CBD/carrier) | ddH₂O | 4 | Yes | 145.7 **A** | 0.264 **A** |

**Table 13**

| **Arm No.:** | **Rats number** | **Administration type** | **Formulation - Stock CBD concentration (mg/mL) and solvent** | **Administration volume (mL) /rat** | **Concentration CBD/rat (mg/kg)** | **Amount CBDZ 200 g rat** |
|---|---|---|---|---|---|---|
| **F1** | 3 | Intravenous CBD | 5 mg/mL CBD in 1:1:20 Ethanol:Tween 80:saline | 200 µl /rat | 5 mg/kg | 1 mg |
| **F2** | 3 | Per oral - Prototype (20 mg/kg) | 4 mg/mL CBD in ddH2O | 1 mL / rat | 20 mg/kg | 4 mg |
| **F3** | 3 | Per oral - Prototype (40 mg/kg) | 4 mg/mL CBD in ddH2O | 2 mL /rat | 40 mg/kg | 8 mg |
| **F4** | 3 | Per oral - Side Batch (40 mg/kg) | 4 mg/mL CBD in ddH2O | 2 mL / rat | 40 mg/kg | 8 mg |
| **F5** | 3 | Per oral Commercial CBD (Epidyolex) | 4 mg/mL CBD in sesame oil | 2 mL / rat | 40 mg/kg | 8 mg |
| **F6** | 3 | Per Oral | DdH2O | 2 mL / rat | - | - |

**Table 14**

| **Batch no.** | **Lipid** | **CBD:lipid ratio** | **Expected CBD:lipid ratio*** | **EE% (for rat study)** | **mg CBD/ g granules** | **Hydrodynamic diameter (d.nm)** | **PDI** | **Zeta Potential (mV)** |
|---|---|---|---|---|---|---|---|---|
| 229717C020 | DMPC | 0.7 | 0.097 | 61 | 61.2 | 512 | 0.749 | - |
| 229717C021 | DMPC | 0.098 | 0.42 | 97 | 9.18 | 116 | 0.095 | - |

**Table 15**

| **Treatment ID** | **Dose** | **Route** | **C0** | **Cmax** | **Cmax_D** | **Tmax** | **Tlast** | **AUClast** | **AUClast_D** | **AUC_inf** | **AUC_extra** | **t1/2** | **Kel** | **Rsq** | **Vd or Vd/F** | **Cl or CLIF** | **F** |
|---|---|---|---|---|---|---|---|---|---|---|---|---|---|---|---|---|---|
| | (mg/kg) | | (ng/mL) | (ng/mL) | (kg*ng/mL/mg) | (hour) | (hour) | (hour*ng/mL) | (hour*kg*ng/mL/mg) | (hour*ng/mL) | (%) | (hour) | (1/hour) | | (L/kg) | (L/h/kg) | % |
| **F1** | 5 | IV | 9351.2 | 6719.4 | 1343.8 | 0.08 | 24 | 5134.1 | 1026.8 | 5569.8 | 7 | 6.5 | 0.112 | 0.732 | 8.3 | 0.9 | |
| **F2** | 20 | PO | 0 | 309.71 | 15.49 | 0.50 | 13.33 | 1465 | 73 | 3097 | 44.15 | 11.63 | 0.08 | 0.83 | 100.91 | 7.54 | 7.13 |
| **F3** | 40 | PO | 0 | 651.28 | 16.28 | 0.50 | 32.00 | 5061 | 127 | 5111 | 1.00 | 4.67 | 0.16 | 0.96 | 56.70 | 8.06 | 12.32 |
| **F4** | 40 | PO | 0 | 1158.2 | 28.96 | 1.83 | 24.00 | 9338 | 233 | 9496 | 2.12 | 4.27 | 0.17 | 0.99 | 31.59 | 4.78 | 22.73 |
| **F5** | 40 | PO | 0 | 363.448 | 9.086 | 6 | 31 | 7147.924 | 178.698 | | | | | | | | 17.56 |
| **Buffer** | 0 | PO | 0 | 0 | 0 | 0 | 0 | 0 | 0 | 0 | 0 | 0 | 0 | 0 | 0 | 0 | 0 |

**Table 16**

| | **C020** | **C021** | **Li196-203** | **Li204-211** |
|---|---|---|---|---|
| Analysis | Fraction(%) | Fraction(%) | Fraction(%) | Fraction(%) |
| Unilamellar liposomes | 48.21 | 58.41 | 32.46 | 18.84 |
| Bilamellar liposomes | 2.00 | 5.39 | 4.22 | 0 |
| Oligolamellar liposomes | 0.89 | 0.63 | 1.29 | 0 |
| Multivesicular liposomes | 13.39 | 16.50 | 9.41 | 10.14 |
| Multilamellar-multivesicular liposomes | 35.49 | 19.04 | 52.59 | 71.01 |

**Table 17**

| **Batch** | **C020** | **C021** | **Li196-203** | **Li204-211** |
|---|---|---|---|---|
| Hydrodynamic diameter (d.nm.) | 981.7 | 158.1 | 501.1 | 145.8 |
| PDI | 0.908 | 0.303 | 0.642 | 0.163 |
| Peak 1 (d.nm.) | 106.9 | 174.7 | 269.8 | 160 |
| Intensity (%) | 68.7 | 93.7 | 68.7 | 99.2 |
| Peak 2 (d.nm.) | 163.1 | 4417 | 1418 | 5064 |
| Intensity(%) | 30.3 | 6.3 | 26.7 | 0.8 |
| Peak 3 (d.nm.) | - | - | 5430 | - |
| Intensity (%) | | | 4.6 | |
| | | | | |

**Table 18**

| **Arm no.** | **Formulation** | **Batch no. (229717...)** | **Vehicle** | **Concentration (mg/mL)** | **Application** |
|---|---|---|---|---|---|
| F1 | Intravenous CBD | - | 5 mg/mL CBD In 1:1:20 Ethanol:Tween 80:Saline | 5 mg/ml CBD | IV at 1 mL/kg |
| F2 | "0.42", without cholesterol | Li297-298 | CBD liposome suspension in 5% Sucrose solution | 4 mg/ml CBD | PO at 10 mL/kg |
| F3 | "0.42", with cholesterol | Li303-304 | | 4 mg/ml CBD | PO at 10 mL/kg |
| F4 | "0.7", without cholesterol | Li299-300 | | 4 mg/ml CBD | PO at 10 mL/kg |
| F5 | "0.7", with cholesterol | Li305-306 | | 4 mg/ml CBD | PO at 10 mL/kg |
| F6 | "1.3", without cholesterol | Li301-302 | | 4 mg/ml CBD | PO at 10 mL/kg |
| F7 | Commercial CBD (Epidyolex) | 229717H001 | 4 mg/mL CBD in sesame oil | 4 mg/ml CBD | PO at 10 mL/kg |

**Table 19**

| **Batch no.** | **Lipid** | **CBD:lipid ratio** | **Expected CBD:lipid ratio*** | **EE% (for rat study)** | **Assay purity (mg CBD/ g dispersion)** | **Hydrodynamic diameter (d.nm)** | **PDI** | **Zeta Potential (mV)** |
|---|---|---|---|---|---|---|---|---|
| 229717Li297-298 | DMPC | 0.42 | 0.39 | 94 | 48.5 | 398,8 | 0,414 | -57,2 |
| 229717Li299-300 | DMPC | 0.7 | 0.63 | 91 | 66.1 | 550,3 | 0,558 | -21,5 |
| 229717Li301-302 | DMPC | 1.3 | 1.13 | 87 | 90.7 | 941,5 | 0,671 | -16,5 |
| 229717Li303-304 | 80%DMPC + 20% Cholesterol | 0.42 | 0.24 | 59 | 40.8 | 399,6 | 0,573 | -11,9 |
| 229717Li305-306 | 80%DMPC + 20% Cholesterol | 0.7 | 0.40 | 58 | 57.7 | 559,2 | 0,727 | -17,3 |

**Table 20**

| **Treatment_ ID** | **Dose** | **Route** | **Subject** | **C0** | **Cmax** | **Cmax_D** | **Tmax** | **Tlast** | **AUClast** | **AUClast_D** | **AUC_inf** | **AUC_ extra** | **t1/2** | **Kel** | **Rsq** | **Vd or Vd/F** | **Cl or CI/F** | **F** |
|---|---|---|---|---|---|---|---|---|---|---|---|---|---|---|---|---|---|---|
| | (mg/ kq) | | | (ng/mL) | (ng/mL) | (kg*ng/mL/ mg) | (hour) | (hour) | (hour*ng/ mL) | (hour*kg*n g/mL/mg) | (hour*ng/m L) | (%) | (hour) | (1/hour) | | (L/kg) | (L/h/kg) | % |
| CBD -F1 | 5 | IV | IV01 | 3350,7 | 2702,1 | 540,42 | 0,08 | 48,00 | 2578,0 | 515,60 | 2659 | 3,06 | 11,25 | 0,06 | 0,80 | 30,50 | 1,88 | |
| | | | IV02 | 3581,7 | 2899,0 | 579,80 | 0,08 | 48,00 | 2454,2 | 490,83 | 2604 | 5,75 | 19,42 | 0,04 | 0,82 | 53,79 | 1,92 | |
| | | | IV03 | 3894,3 | 3122,1 | 624,42 | 0,08 | 48,00 | 2500,6 | 500,12 | 2732 | 8,47 | 31,92 | 0,02 | 0,97 | 84,28 | 1,83 | |
| | | | N | 3 | 3 | 3 | 3 | 3 | 3 | 3 | 3 | 3 | 3 | 3 | 3 | 3 | 3 | |
| | | | **Mean** | **3608,897** | **2907,7** | **581,55** | **0,08** | **48,00** | **2510,9** | **502,19** | **2665** | **5,76** | **20,86** | **0,04** | **0,86** | **56,19** | **1,88** | |
| | | | SD | 272,79 | 210,14 | 42,03 | 0,00 | 0,00 | 62,563 | 12,51 | 64 | 2,70 | 10,41 | 0,02 | 0,09 | 26,97 | 0,05 | |
| | | | CV% | 7,6 | 7,2 | 7,2 | 0,0 | 0,0 | 2,5 | 2,5 | 2,4 | 46,9 | 49,9 | 51,0 | 10,3 | 48,0 | 2,4 | |
| | | | Min | 3350,7 | 2702,1 | 540,42 | 0,08 | 48,00 | 2454,2 | 490,83 | 2604 | 3,06 | 11,25 | 0,02 | 0,80 | 30,50 | 1,83 | |
| | | | Median | 3581,7 | 2899,0 | 579,80 | 0,08 | 48,00 | 2500,6 | 500,12 | 2659 | 5,75 | 19,42 | 0,04 | 0,82 | 53,79 | 1,88 | |
| | | | Max | 3894,3 | 3122,1 | 624,42 | 0,08 | 48,00 | 2578,0 | 515,60 | 2732 | 8,47 | 31,92 | 0,06 | 0,97 | 84, 28 | 1,92 | |
| | | | Geometric Mean | 3602,1 | 2902,7 | 580,54 | 008 | 48,00 | 2510,4 | 502,08 | 2665 | 5,30 | 19,10 | 0,04 | 0,86 | 51,71 | 1,88 | |

**Table 21**

| **Treatment _ID** | **Dose** | **Route** | **Subject** | **C0** | **Cmax** | **Cmax_D** | **Tmax** | **Tlast** | **AUClast** | **AUClast_ D** | **AUC_inf** | **AUC_ extra** | **t1/2** | **Kel** | **Rsq** | **Vd or Vd/F** | **CI or CI/F** | **F** |
|---|---|---|---|---|---|---|---|---|---|---|---|---|---|---|---|---|---|---|
| | (mg/ kg) | | | (ng/ mL) | (ng/mL) | (kg*ng/ mL/mg) | (hour) | (hour) | (hour*ng/ mL) | (hour*kg* ng/mL/mg) | (hour*ng/ mL) | (%) | (hour) | (1/hour) | | (L/kg) | (L/h/kg) | % |
| CBD -F2 | 40 | PO | PO04 | | 835,05 | 20,88 | 4,00 | 48,00 | 11211 | 280,28 | 11313 | 0,90 | 7,01 | 0,10 | 0,87 | 35,75 | 3,54 | 55,81 |
| | | | PO05 | | 1243,8 | 31,10 | 0,50 | 48,00 | 12387 | 309,69 | 12465 | 0,62 | 6,24 | 0,11 | 0,90 | 28,89 | 3,21 | 61,67 |
| | | | PO06 | | 759,03 | 18,98 | 0,50 | 48,00 | 7034,7 | 175,87 | 7095 | 0,85 | 6,43 | 0,11 | 0,92 | 52,30 | 5,64 | 35,02 |
| | | | N | 0 | 3 | 3 | 3 | 3 | 3 | 3 | 3 | 3 | 3 | 3 | 3 | 3 | 3 | |
| | | | **Mean** | | **945,96** | **23,65** | **1,67** | **48,00** | **10211** | **255,28** | **10291** | **0,79** | **6,56** | **0,11** | **0,89** | **38,98** | **4,13** | **50,83** |
| | | | SD | | 260,72 | 6,52 | 2,02 | 0,00 | 2813,1 | 70,33 | 2827 | 0,15 | 0,40 | 0,01 | 0,03 | 12,04 | 1,32 | 14,00 |
| | | | CV% | | 27,6 | 27,6 | 121,2 | 0,0 | 27,5 | 27,5 | 27,5 | 18,5 | 6,1 | 5,9 | 2,9 | 30,9 | 31,9 | |
| | | | Min | | 759,03 | 18,98 | 0,50 | 48,00 | 7034,7 | 175,87 | 7095 | 0,62 | 6,24 | 0,10 | 0,87 | 28,89 | 3,21 | |
| | | | Median | | 835,05 | 20,88 | 0,50 | 48,00 | 11211 | 280,28 | 11313 | 0,85 | 6,43 | 0,11 | 0,90 | 35,75 | 3,54 | |
| | | | Max | | 1243,8 | 31,10 | 4,00 | 48,00 | 12387 | 309,69 | 12465 | 0,90 | 7,01 | 0,11 | 0,92 | 52,30 | 5,64 | |
| | | | Geometric Mean | | 923,79 | 23,10 | 1,00 | 48,00 | 9922,7 | 248,07 | 10002 | 0,78 | 6,55 | 0,11 | 0,89 | 37,80 | 4,00 | |
| CBD -F3 | 40 | PO | PO07 | | 1873,2 | 46,83 | 4,00 | 48,00 | 18858 | 471,45 | 18952 | 0,49 | 6,28 | 0,11 | 0,84 | 19,12 | 2,11 | 93,88 |
| | | | PO08 | | 1579,6 | 39,49 | 2,00 | 48,00 | 13424 | 335,60 | 13531 | 0,79 | 6,63 | 0,10 | 0,92 | 28,26 | 2,96 | 66,83 |
| | | | PO09 | | 915,21 | 22,88 | 0,50 | 48,00 | 6945,5 | 173,64 | 7014 | 0,98 | 6,57 | 0,11 | 0,93 | 54,08 | 5,70 | 34,58 |
| | | | N | 0 | 3 | 3 | 3 | 3 | 3 | 3 | 3 | 3 | 3 | 3 | 3 | 3 | 3 | |
| | | | **Mean** | | **1456,0** | **36,40** | **2,17** | **48,00** | **13076** | **326,90** | **13166** | **0,76** | **6,49** | **0,11** | **0,90** | **33,82** | **3,59** | **65,09** |
| | | | SD | | 490,81 | 12,27 | 1,76 | 0,00 | 5963,8 | 149,10 | 5977 | 0,25 | 0,19 | 0,00 | 0,05 | 18,13 | 1,88 | 29,69 |
| | | | CV% | | 33,7 | 33,7 | 81,0 | 0,0 | 45,6 | 45,6 | 45,4 | 32,5 | 2,9 | 2,9 | 5,3 | 53,6 | 52,3 | |
| | | | Min | | 915,21 | 22,88 | 0,50 | 48,00 | 6945,5 | 173,64 | 7014 | 0,49 | 6,28 | 0,10 | 0,84 | 19,12 | 2,11 | |
| | | | Median | | 1579,6 | 39,49 | 2,00 | 48,00 | 13424 | 335,60 | 13531 | 0,79 | 6,57 | 0,11 | 0,92 | 28,26 | 2,96 | |
| | | | Max | | 1873,2 | 46,83 | 4,00 | 48,00 | 18858 | 471,45 | 18952 | 0,98 | 6,63 | 0,11 | 0,93 | 54,08 | 5,70 | |
| | | | Geometric Mean | | 1393,9 | 34,85 | 1,59 | 48,00 | 12070 | 301,74 | 12161 | 0,73 | 6,49 | 0,11 | 0,90 | 30,80 | 3,29 | |

**Table 22**

| **Treatment _ID** | **Dose** | **Route** | **Subject** | **C0** | **Cmax** | **Cmax_D** | **Tmax** | **Tlast** | **AUClast** | **AUClast_ D** | **AUC_inf** | **AUC_ extra** | **t1/2** | **Kel** | **Rsq** | **Vd or Vd/F** | **CI or CI/F** | **F** |
|---|---|---|---|---|---|---|---|---|---|---|---|---|---|---|---|---|---|---|
| | (mg/ kg) | | | (ng/ mL) | (ng/mL) | (kg*ng/ mL/mg) ql | (hour) | (hour) | (hour*ng/ mL) | (hour*kg*n g/mL/mg) | (hour*ng/ mL) | (%) | (hour) | (1/hour) | | (L/kg) | (L/h/kg) | % |
| CBD -F4 | 40 | PO | PO10 | | 1044,6 | 26,12 | 0,50 | 48,00 | 13850 | 346,26 | 13942 | 0,66 | 6,48 | 0,11 | 0,91 | 26,83 | 2,87 | 68,95 |
| | | | PO11 | | 2128,2 | 53,21 | 2,00 | 48,00 | 18186 | 454,65 | 18347 | 0,88 | 6,65 | 0,10 | 0,85 | 20,91 | 2,18 | 90,53 |
| | | | PO12 | | 1621,4 | 40,54 | 4,00 | 48,00 | 17968 | 449,21 | 18084 | 0,64 | 6,69 | 0,10 | 0,92 | 21,36 | 2,21 | 89,45 |
| | | | N | 0 | 3 | 3 | 3 | 3 | 3 | 3 | 3 | 3 | 3 | 3 | 3 | 3 | 3 | |
| | | | **Mean** | | **1598,1** | **39,95** | **2,17** | **48,00** | **16668** | **416,70** | **16791** | **0,73** | **6,61** | **0,11** | **0,90** | **23,03** | **2,42** | **82,98** |
| | | | SD | | 542,18 | 13,55 | 1,76 | 0,00 | 2442,7 | 61,07 | 2471 | 0,13 | 0,11 | 0,00 | 0,04 | 3,30 | 0,39 | 12,16 |
| | | | CV% | | 33,9 | 33,9 | 81,0 | 0,0 | 14,7 | 14,7 | 14,7 | 18,5 | 1,7 | 1,7 | 4,4 | 14,3 | 16,1 | |
| | | | Min | | 1044,6 | 26,12 | 0,50 | 48,00 | 13850 | 346,26 | 13942 | 0,64 | 6,48 | 0,10 | 0,85 | 20,91 | 2,18 | |
| | | | Median | | 1621,4 | 40,54 | 2,00 | 48,00 | 17968 | 449,21 | 18084 | 0,66 | 6,65 | 0,10 | 0,91 | 21,36 | 2,21 | |
| | | | Max | | 2128,2 | 53,21 | 4,00 | 48,00 | 18186 | 454,65 | 18347 | 0,88 | 6,69 | 0,11 | 0,92 | 26,83 | 2,87 | |
| | | | Geometric Mean | | 1533,3 | 38,33 | 1,59 | 48,00 | 16541 | 413,53 | 16662 | 0,72 | 6,61 | 0,11 | 0,89 | 22,88 | 2,40 | |
| CBD -F5 | 40 | PO | PO13 | | 1381,4 | 34,54 | 2,00 | 48,00 | 9030,9 | 225,77 | 9098 | 0,74 | 6,40 | 0,11 | 0,83 | 40,59 | 4,40 | 44,96 |
| | | | PO14 | | 1734,9 | 43,37 | 2,00 | 48,00 | 13185 | 329,62 | 13287 | 0,77 | 6,60 | 0,11 | 0,93 | 28,65 | 3,01 | 65,64 |
| | | | PO15 | | 1497,2 | 37,43 | 4,00 | 48,00 | 19236 | 480,91 | 19359 | 0,63 | 6,55 | 0,11 | 0,85 | 19,53 | 2,07 | 95,76 |
| | | | N | 0 | 3 | 3 | 3 | 3 | 3 | 3 | 3 | 3 | 3 | 3 | 3 | 3 | 3 | |
| | | | **Mean** | | **1537,8** | **38,45** | **2,67** | **48,00** | **13817** | **345,43** | **13915** | **0,71** | **6,52** | **0,11** | **0,87** | **29,59** | **3,16** | **68,79** |
| | | | SD | | 180,22 | 4,51 | 1,16 | 0,00 | 5132,0 | 128,30 | 5159 | 0,07 | 0,10 | 0,00 | 0,05 | 10,56 | 1,17 | 25,55 |
| | | | CV% | | 11,7 | 11,7 | 43,3 | 0,0 | 37,1 | 37,1 | 37,1 | 9,9 | 1,6 | 1,6 | 6,1 | 35,7 | 37,1 | |
| | | | Min | | 1381,4 | 34,54 | 2,00 | 48,00 | 9030,9 | 225,77 | 9098 | 0,63 | 6,40 | 0,11 | 0,83 | 19,53 | 2,07 | |
| | | | Median | | 1497,2 | 37,43 | 2,00 | 48,00 | 13185 | 329,62 | 13287 | 0,74 | 6,55 | 0,11 | 0,85 | 28,65 | 3,01 | |
| | | | Max | | 1734,9 | 43,37 | 4,00 | 48,00 | 19236 | 480,91 | 19359 | 0,77 | 6,60 | 0,11 | 0,93 | 40,59 | 4,40 | |
| | | | Geometric Mean | | 1530,9 | 38,27 | 2,52 | 48,00 | 13182 | 329,54 | 13277 | 0,71 | 6,51 | 0,11 | 0,87 | 28,32 | 3,01 | |

**Table 23**

| **Treatment _ID** | **Dose** | **Route** | **Subject** | **C0** | **Cmax** | **Cmax_ D** | **Tmax** | **Tlast** | **AUClast** | **AUClast_D** | **AUC_in f** | **AUC_ extra** | **t1/2** | **Kel** | **Rsq** | **Vd or Vd/F** | **CI or CI/F** | **F** |
|---|---|---|---|---|---|---|---|---|---|---|---|---|---|---|---|---|---|---|
| | (mg/ kg) | | | (ng/ mL) | (ng/mL) | (kg*ng/ mL/mg) | (hour) | (hour) | (hour*ng/ mL) | (hour*kg* ng/mL/mg) | (hour* ng/mL) | (%) | (hour) | (1/hour) | | (L/kg) | (L/h/kg) | % |
| CBD -F6 | 40 | PO | PO16 | | 1082,5 | 27,06 | 2,00 | 48,00 | 15018 | 375,45 | 15153 | 0,89 | 6,88 | 0,10 | 0,96 | 26,19 | 2,64 | 74,76 |
| | | | PO17 | | 1116,9 | 27,92 | 4,00 | 48,00 | 14292 | 357,31 | 14434 | 0,98 | 7,28 | 0,10 | 0,80 | 29,09 | 2,77 | 71,15 |
| | | | PO18 | | 1046,8 | 26,17 | 2,00 | 48,00 | 10875 | 271,87 | 10992 | 1,07 | 7,14 | 0,10 | 0,91 | 37,50 | 3, 64 | 54,14 |
| | | | N | 0 | 3 | 3 | 3 | 3 | 3 | 3 | 3 | 3 | 3 | 3 | 3 | 3 | 3 | |
| | | | **Mean** | | **1082,1** | **27,05** | **2,67** | **48,00** | **13395** | **334,88** | **13527** | **0,98** | **7,10** | **0,10** | **0,89** | **30,93** | **3,02** | **66,68** |
| | | | SD | | 35,052 | 0,88 | 1,16 | 0,00 | 2212,6 | 55,32 | 2224 | 0,09 | 0,20 | 0,00 | 0,09 | 5,87 | 0,54 | 11,01 |
| | | | CV% | | 3,2 | 3,2 | 43,3 | 0,0 | 16,5 | 16,5 | 16,4 | 9,0 | 2,9 | 2,9 | 9,6 | 19,0 | 18,0 | |
| | | | Min | | 1046,8 | 26,17 | 2,00 | 48,00 | 10875 | 271,87 | 10992 | 0,89 | 6,88 | 0,10 | 0,80 | 26,19 | 2,64 | |
| | | | Median | | 1082,5 | 27,06 | 2,00 | 48,00 | 14292 | 357,31 | 14434 | 0,98 | 7,14 | 0,10 | 0,91 | 29,09 | 2,77 | |
| | | | Max | | 1116,9 | 27,92 | 4,00 | 48,00 | 15018 | 375,45 | 15153 | 1,07 | 7,28 | 0,10 | 0,96 | 37,50 | 3,64 | |
| | | | Geometric Mean | | 1081,7 | 27,04 | 2,52 | 48,00 | 13265 | 331,63 | 13397 | 0,98 | 7,10 | 0,10 | 0,89 | 30,57 | 2,99 | |
| CBD -F7 | 40 | PO | PO19 | | 547,03 | 13,68 | 4,00 | 48,00 | 6569,7 | 164,24 | 6727 | 2,34 | 8,6 | 0,08 | 0,97 | 73,81 | 5,95 | 32,71 |
| | | | PO20 | | 421,26 | 10,53 | 4,00 | 48,00 | 6557,9 | 163,95 | 6875 | 4,61 | 10,22 | 0,07 | 0,96 | 85,77 | 5,82 | 32,65 |
| | | | PO21 | | 413,07 | 10,33 | 4,00 | 48,00 | 6066,7 | 151,67 | 6261 | 3,10 | 9,42 | 0,07 | 1 | 86,87 | 6,39 | 30,20 |
| | | | N | 0 | 3 | 3 | 3 | 3 | 3 | 3 | 3 | 3 | 3 | 3 | 3 | 3 | 3 | |
| | | | **Mean** | | **460,45** | **11,51** | **4,00** | **48,00** | **6398,1** | **159,95** | **6621** | **3,35** | **9,42** | **0,07** | **0,98** | **82,15** | **6,05** | **31,85** |
| | | | SD | | 75,089 | 1,88 | 0,00 | 0,00 | 287,05 | 7,18 | 321 | 1,16 | 0,81 | 0,01 | 0,02 | 7,24 | 0,30 | 1,43 |
| | | | CV% | | 16,3 | 16,3 | 0,0 | 0,0 | 4,5 | 4,5 | 4,8 | 34,5 | 8,6 | 8,6 | 2,1 | 8,8 | 4,9 | |
| | | | Min | | 413,07 | 10,33 | 4,00 | 48,00 | 6066,7 | 151,67 | 6261 | 2,34 | 8,60 | 0,07 | 0,96 | 73,81 | 5,82 | |
| | | | Median | | 421,26 | 10,53 | 4,00 | 48,00 | 6557,9 | 163,95 | 6727 | 3,10 | 9,42 | 0,07 | 0,97 | 85,77 | 5,95 | |
| | | | Max | | 547,03 | 13,68 | 4,00 | 48,00 | 6569,7 | 164,24 | 6875 | 4,61 | 10,22 | 0,08 | 1,00 | 86,87 | 6,39 | |
| | | | Geometric Mean | | 456,59 | 11,42 | 4,00 | 48,00 | 6393,7 | 159,84 | 6616 | 3,22 | 9,39 | 0,07 | 0,98 | 81,93 | 6,05 | |

**Table 24**

| **Batch 229717...** | **C018** | **C019** |
|---|---|---|
| Used liposome dispersion | LN001 | LN002 |
| Dispersing agent | ddH₂O + 5% Saccharose | ddH₂O + 5% Saccharose |
| CBD concentration of sprayed dispersion [mg/ml] | 9.00 | 9.00 |
| Lipid concentration of sprayed dispersion [mg/ml] | 91.00 | 91.00 |
| Final theoretical amount CBD per g carrier [mg/g] | - | 12.01 |
| Final amount lipid per g carrier [mg/g] | - | 121.41 |
| Batch size [g Isomalt 720] | 390 | 742.5 |
| Hydrodynamic diameter d_{H} (nm) | 339.9 **C** | 748.4 **C** |
| PDI | 0.81 **C** | 0.946 **C** |
| Attenuator | 10 | 10 |

## Claims

1. A liposome comprising cannabidiol (CBD), a phospholipid component and optionally a sterol component, wherein the liposome comprises more than one bilayer.

2. The liposome according to claim 1, which fulfils one of the following conditions:
a) the phospholipid component comprises or consists of phosphatidylcholine, wherein the phosphatidylcholine is preferably 1,2-dimyristoyl-sn-glycero-3-phosphocholine (DMPC),
b) the liposome comprises a sterol component which comprises or consists of cholesterol,
c) the liposome does not comprise a sterol component,
d) the ratio of the weight of CBD to the total weight of the phospholipid component and, if present, the sterol component (CBD:lipid ratio) is 0.25, 0.42, 0.5, 0.7, 0.8, 1.0, 1.3, from 0.1 to 9, from 0.1 to 4, from 0.1 to 2.3, from 0.1 to 1.3, from 0.25 to 1.3, from 0.42 to 1.0 or from 0.5 to 0.8, wherein the CBD:lipid ratio is preferably 0.7,
e) the liposome is substantially free from tetrahydrocannabinol,
f) a combination of the above a) and b),
g) a combination of the above a) and b), wherein the weight ratio of the phospholipid component to the sterol component is preferably from 70:30 to 90:10, more preferably from 75:25 to 85:15 and most preferably 80:20,
h) a combination of the above a) and c),
i) a combination of the above a), b) and d),
j) a combination of the above a), c) and d),
k) a combination of the above a), b), d) and e), or
l) a combination of the above a), c), d) and e).

3. The liposome according to claim 1 or 2, which is selected from the group consisting of a bilamellar liposome (BLV), an oligolamellar liposome (OLV), a multilamellar liposome (MLV), a multivesicular liposome (MVV) and a multilamellar-multivesicular liposome (MLV-MVV).

4. A mixture of liposomes, wherein the liposomes comprise cannabidiol (CBD), a phospholipid component and optionally a sterol component, and wherein the mixture fulfils one of the following conditions:
a) the proportion by number of multilamellar-multivesicular liposomes (MLV-MVV) in the mixture is at least 10%,
b) the proportion by number of multivesicular liposomes (MVV) in the mixture is at least 5%,
c) the proportion by number of unilamellar liposomes (ULV) in the mixture is at most 70%,
d) the Z-average hydrodynamic diameter, assayed by dynamic light scattering, is greater than 100 nm,
e) the polydispersity index (PDI), assayed by dynamic light scattering, is greater than 0.15,
f) a combination of the above a) and b),
g) a combination of the above a) and c),
h) a combination of the above b) and c),
i) a combination of the above a), b) and c),
j) a combination of the above a) and d),
k) a combination of the above a) and e),
l) a combination of the above a), d) and e),
m) a combination of the above d) and e), or
n) a combination of the above a), b), c), d) and e).

5. The mixture according to claim 4, wherein the liposomes fulfil one of the following conditions:
a) the phospholipid component comprises or consists of phosphatidylcholine, wherein the phosphatidylcholine is preferably 1,2-dimyristoyl-sn-glycero-3-phosphocholine (DMPC),
b) the liposomes comprise a sterol component which comprises or consists of cholesterol,
c) the liposomes do not comprise a sterol component,
d) the ratio of the weight of CBD to the total weight of the phospholipid component and, if present, the sterol component (CBD:lipid ratio) is 0.25, 0.42, 0.5, 0.7, 0.8, 1.0, 1.3, from 0.1 to 9, from 0.1 to 4, from 0.1 to 2.3, from 0.1 to 1.3, from 0.25 to 1.3, from 0.42 to 1.0 or from 0.5 to 0.8, wherein the CBD:lipid ratio is preferably 0.7,
e) the liposomes are substantially free from tetrahydrocannabinol,
f) a combination of the above a) and b),
g) a combination of the above a) and b), wherein the weight ratio of the phospholipid component to the sterol component is preferably from 70:30 to 90:10, more preferably from 75:25 to 85:15 and most preferably 80:20,
h) a combination of the above a) and c),
i) a combination of the above a), b) and d),
j) a combination of the above a), c) and d),
k) a combination of the above a), b), d) and e), or
l) a combination of the above a), c), d) and e).

6. The mixture according to claim 4 or 5, which has a bioavailability of at least 25%, when assayed in Sprague-Dawley rats at a concentration of 40 mg CBD per kg of body weight in peroral administration.

7. The mixture according to any of claims 4 to 6, which has not been subjected to spray-drying.

8. A process for manufacturing liposomes comprising cannabidiol (CBD), comprising the following steps:
a) providing CBD, a phospholipid component and optionally a sterol component,
b) providing beads, preferably ceramic beads, more preferably ceramic beads made of zirconium oxide / yttrium stabilised,
c) providing a liquid, preferably selected from the group consisting of water and 5% sucrose in water,
d) providing a vessel with a combination of the beads, the liquid, the CBD, the phospholipid component and, if present, the sterol component,
e) a first step of centrifuging the vessel with the combination in a centrifuge with a rotor which rotates the vessel around two different axes,
f) optionally adding a first additional volume of the liquid to the vessel,
g) a second step of centrifuging the vessel in the centrifuge with the rotor,
h) optionally adding a second additional volume of the liquid to the vessel.

9. The process according to claim 8, which fulfils one of the following conditions:
a) the beads have a diameter of 0.8 mm,
b) the beads have a diameter of 1.4 to 1.6 mm,
c) the vessel has an internal volume of 1.5 to 50 ml, preferably 2 to 10 ml,
d) the vessel has an internal volume of 2 ml and is preferably a 2 ml vial,
e) the vessel has an internal volume of 10 ml and is preferably a 10 ml injection flask,
f) the rotor has a first rotation unit and second rotation units, and the vessel is preferably contacted with a second rotation unit such that it is oriented horizontally in relation to the plane of the second rotation unit and/or such that a short axis of the vial is in the same orientation as the axis of rotation of the second rotation unit,
g) a combination of the above a) and e), or
h) a combination of the above b) and d)

10. A process for manufacturing liposomes comprising cannabidiol (CBD), comprising the following steps:
a) providing CBD, a phospholipid component and optionally a sterol component,
b) providing beads, preferably ceramic beads, more preferably ceramic beads made of zirconium oxide / yttrium stabilised,
c) providing a liquid, preferably selected from the group consisting of water and 5% sucrose in water,
d) an optional preliminary homogenising step of the room temperature liquid together with the phospholipid component and, if present, the sterol component in a disperser,
e) a homogenising step of a combination of the liquid, the CBD, the phospholipid component and, if present, the sterol component, and
f) milling the homogenised combination together with the beads in a bead mill having a grinding chamber.

11. The process according to claim 10, which fulfils one of the following conditions:
a) the beads have a diameter of 0.3 to 0.8 mm, preferably 0.5 to 0.8 mm, more preferably 0.5 mm,
b) the total milling time is 300 min or greater, in particular 330 min,
c) the grinding chamber has been filled with the beads to 50% or greater of its working volume, preferably to 75%,
d) the volume of the combination is 600 to 1200 ml,
e) the maximum agitator speed is 2100 to 2500 rpm, in particular 2150 rpm,
f) the pump speed is 160 to 200 rpm, in particular 165 rpm,
g) a combination of the above a) and b), or
h) a combination of the above a) to e).

12. Liposomes manufactured by the process of any of claims 8 to 11.

13. The
- liposome according to any of claims 1 to 3,
- mixture according to any of claims 4 to 7 or
- liposomes according to claim 12
for use as a medicament.

14. The
- liposome according to any of claims 1 to 3,
- mixture according to any of claims 4 to 7 or
- liposomes according to claim 12
for use in the treatment of a condition selected from the group consisting of a sleeping disorder, anxiety, depression, a pain disorder, a neurological disorder and spasticity, wherein the liposome, mixture or liposomes are preferably administered orally.

15. A pharmaceutical composition comprising the liposome according to any of claims 1 to 3, the mixture according to any of claims 4 to 7 or the liposomes according to claim 12, and a pharmaceutically acceptable excipient.

16. A capsule, liquid, gel, tablet or stick pack comprising the liposome according to any of claims 1 to 3, the mixture according to any of claims 4 to 7, the liposomes according to claim 12 or the pharmaceutical composition according to claim 15.
